# FASCICULE DE BREVET EUROPEEN

(11) **EP 4 087 496 B1**
(45) Date de publication et mention de la délivrance du brevet: **26.06.2024**
(21) Numéro de dépôt: 21708656.0
(22) Date de dépôt: 03.03.2021
(51) Int. Cl.: A61B 10/00, A61B 5/145, G01N 33/493, A61B 5/20, A61B 5/00, G01N 33/487

(54) **DISPOSITIF ET MÉTHODE D'ANALYSE D'URINE**
URINANALYSEVERFAHREN UND -VORRICHTUNG
URINE ANALYSIS METHOD AND DEVICE

(30) Priorité: 03.03.2020 FR 2002124
(43) Date de publication de la demande: 16.11.2022
(73) Titulaire: Withings, 92130 Issy-les-Moulineaux (FR)
(72) Inventeur: BARBEDETTE, Thibaut, 92130 ISSY LES MOULINEAUX (FR); KIRAT, Marine, 92130 ISSY LES MOULINEAUX (FR); LOY, Jonas, 92130 ISSY LES MOULINEAUX (FR); TUCOULAT, Benoît, 92130 ISSY LES MOULINEAUX (FR); DEWAVRIN, Julius, 92130 ISSY LES MOULINEAUX (FR)
(74) Mandataire: Withings IP
(86) Numéro de dépôt international: PCT/EP2021/055302
(87) Numéro de publication internationale: WO 2021/175909

(56) Documents cités:
- EP-A1- 0 278 631
- WO-A2-2010/094427
- CN-U- 202 676 717
- CN-U- 206 052 899
- US-A1- 2006 064 035
- US-A1- 2015 140 681
- US-A1- 2018 055 488
- US-A1- 2018 372 717
- US-B1- 9 810 686
- US-B1- 10 383 606

## Description

### Domaine technique

La présente divulgation relève du domaine des dispositifs d'analyse d'urine destinés à être positionnés à l'intérieur de toilettes. La présente divulgation vise également une méthode pour analyser l'urine étant reçue dans des toilettes.

### Technique antérieure

De nombreux paramètres biologiques sont reflétés dans l'urine d'un individu. A partir d'un échantillon d'urine, il est par exemple possible de détecter des problèmes de santé tels qu'une infection urinaire, le diabète ou une insuffisance rénale. L'échantillon d'urine peut également refléter la qualité d'une alimentation, identifier une période de fécondité ou une grossesse et détecter une prise de drogues ou de tabac. Il est alors intéressant de surveiller divers paramètres biologiques périodiquement.

Il est connu de proposer des dispositifs installés dans des toilettes ayant une fonction d'analyse d'urine. Ces dispositifs sont capables d'effectuer des prélèvements d'échantillons d'urine depuis les toilettes et de les analyser afin de déterminer le niveau d'un paramètre biologique.

On connait du document US20180188231 un dispositif d'analyse d'urine à fixer sur un rebord de toilettes. Ce dispositif permet de réaliser une analyse avec un transistor à effet de champ.

Il est proposé dans les documents US20170284925 et US10383606 des dispositifs comportant des bandelettes de test défilant devant une section d'analyse. Le document CN206052899 décrit des toilettes intelligentes et un dispositif selon le préambule de la revendication 1; le document US9810686 décrit une cassette d'analyse d'urine ; le document US10383606 décrit un système d'analyse d'urine couplé à des toilettes ; le document EP0278631 décrit des toilettes avec un système d'examen de santé.

Cependant, de tels dispositifs sont volumineux. Notamment, ils nécessitent une zone de stockage de bandelettes de test neuves et de bandelettes de tests usagées. Ces dispositifs doivent alors être positionnés en grande partie à l'extérieur des toilettes ou doivent être intégrés à celles-ci.

Par ailleurs, de tels dispositifs ne sont pas flexibles. Il apparait particulièrement difficile de recharger des bandelettes de test neuves et de retirer des bandelettes usagées. Il apparait également difficile de réaliser des analyses nécessitant plusieurs types de bandelettes.

Il existe donc un besoin pour un dispositif d'analyse d'urine ne présentant pas les inconvénients de l'art antérieur.

**Exposé de l'invention**

La présente description vise à proposer une ou plusieurs solutions résolvant au moins certains des inconvénients précités. L'invention est définie dans les revendications.

Dans un mode de réalisation, il est proposé un dispositif d'analyse d'urine destiné à être positionné à l'intérieur de toilettes comprenant un ensemble de test comportant :
- au moins un support rotatif comprenant une pluralité de bandelettes de test fixées sur le support rotatif ;
- un injecteur configuré pour injecter un volume contrôlé d'urine sur au moins une des bandelettes de test ;
- un système d'analyse configuré pour détecter un résultat d'injection d'urine sur la bandelette de test dans lequel l'injecteur comporte une seringue mobile configurée pour être rapprochée ou éloignée d'une bandelette de test.

Ainsi, avantageusement, le dispositif d'analyse d'urine est suffisamment compact pour être entièrement positionné à l'intérieur des toilettes. Il peut alors être discret et être installé et retiré facilement. Il peut également s'adapter à tout type de toilettes. L'emploi d'un support rotatif sur lequel sont fixées les bandelettes permet de réaliser une pluralité d'analyses de façon très simple. L'utilisation d'un tel support rotatif permet de réduire l'encombrement du dispositif pour un nombre d'analyses donné. Le support rotatif peut en outre être remplacé. Le dispositif est ainsi modulable et polyvalent.

Par *'support rotatif',* il faut comprendre ici que le support est une pièce montée à rotation sur une pièce formant embase. On utilisera le terme de station pour désigner le dispositif d'analyse d'urine sans le support rotatif (la pièce formant embase faisant donc partie de la station). On utilisera aussi indifféremment le terme de cartouche pour désigner le support rotatif, étant donné que ce dernier est un consommable pouvant être remplacé. La station et la cartouche forment deux entités distinctes qui peuvent être fabriquées et vendues indépendamment l'une de l'autre.

Dans un mode de réalisation, il est aussi proposé une cartouche pour dispositif d'analyse d'urine (qui sera donc indifféremment appelée cartouche ou support rotatif), la cartouche étant configurée pour être montée à rotation sur la station (par exemple une embase du dispositif d'urine) du dispositif d'analyse d'urine tel que décrit précédemment, la cartouche comprenant une pluralité de bandelettes de test, fixées sur la cartouche.

Dans un mode de réalisation, il est aussi proposé une station pour dispositif d'analyse d'urine tel que décrit précédemment. La station est configurée pour recevoir une cartouche montée à rotation et comprenant une pluralité de bandelettes de test. La station comprend typiquement un injecteur configuré pour injecter un volume contrôlé d'urine sur au moins une des bandelettes de test et un système d'analyse configuré pour détecter un résultat d'injection d'urine sur la bandelette de test.

Dans un mode de réalisation, il est aussi proposé un kit comprenant une station telle que présentée précédemment et au moins une cartouche telle que présentée précédemment. En particulier, un kit peut comprendre deux cartouches et les deux cartouches peuvent avoir une configuration de bandelettes différente l'une de l'autre (pour mesurer différents composés).

Les caractéristiques exposées dans les paragraphes suivants peuvent, optionnellement, être mises en oeuvre. Elles peuvent être mises en oeuvre indépendamment les unes des autres ou en combinaison les unes avec les autres.

Le ou chaque support rotatif peut être configuré pour entrainer en rotation une bandelette de test et la présenter sélectivement devant l'injecteur et/ou devant le système d'analyse. Moyennant quoi, le nombre de pièces mobiles du dispositif est réduit. Il apparait également possible de sélectionner un type de bandelette précise pour réaliser une analyse. Le dispositif est polyvalent et modulable.

Le ou chaque support rotatif peut être configuré pour tourner selon la direction horaire et antihoraire. Ainsi, la bandelette de test peut être présentée devant l'injecteur et/ou le système d'analyse selon la trajectoire la plus courte. Cette disposition permet aussi une grande flexibilité dans la conception du dispositif, en réduisant notamment les contraintes quant au positionnement du système d'analyse ou de l'injecteur par rapport au support rotatif.

Le ou chaque support rotatif peut comporter des logements recevant une ou plusieurs bandelettes de test. Ainsi, les bandelettes de test sont maintenues dans le support rotatif. Chaque logement peut séparer une ou plusieurs bandelettes de test de bandelettes de test voisines, ce qui améliore la conservation des bandelettes et la précision de l'analyse. Les bandelettes sont fixées dans leur logement de façon à ne pas bouger dudit logement durant une utilisation normale du support rotatif.

Les logements peuvent être positionnés le long d'un cercle ou d'une portion de cercle, à équidistance de l'axe de rotation du support rotatif. En particulier, les logements sont disposés parallèlement les uns aux autres, et, plus spécifiquement, parallèlement à l'axe de rotation du support rotatif. Le support rotatif est alors essentiellement invariant par incrément de rotation (au type de bandelettes près).

Les logements peuvent être fermés par au moins un opercule, par exemple transparent ou translucide. Ainsi, des réactifs contenus dans les bandelettes de test sont préservés avant une analyse. Elles peuvent être analysées facilement par le système d'analyse, notamment par analyse optique.

Le support rotatif peut être cylindrique, et les logements peuvent être agencés sur une paroi externe du support rotatif. Cette configuration permet de maximiser le nombre de bandelettes de test reçues dans les logements du support rotatif. Un nombre important d'analyses peut être réalisé sans nécessiter de recharger le dispositif en bandelettes de test.

Dans une variante, les logements peuvent être ménagés sur une paroi interne du support rotatif cylindrique. Cette disposition permet une grande flexibilité dans la conception du dispositif, en réduisant notamment les contraintes quant au positionnement du système d'analyse ou de l'injecteur par rapport au support rotatif.

De plus, dans cette disposition, l'injecteur et/ou au moins une partie du système d'analyse peuvent être agencés dans une zone radialement interne du support rotatif. Ceci permet de maximiser le diamètre du support rotatif sans nécessiter une augmentation de la taille du boitier. On maximise ainsi le nombre de bandelettes de test logeables et donc le nombre d'analyses disponibles.

L'injecteur comporte une seringue mobile configurée pour être rapprochée ou éloignée d'une bandelette de test. A cet effet, la seringue peut être agencée sur un moteur linéaire. Le moteur linéaire est monté sur la station.

Les bandelettes peuvent être positionnées selon un cercle ou une portion de cercle, à équidistance de l'axe de rotation du support rotatif. En particulier, les bandelettes peuvent être disposées parallèlement les unes aux autres, et, plus spécifiquement, parallèlement à l'axe de rotation du support rotatif, ce qui permet d'agencer un nombre élevé de bandelettes par unité angulaire.

Le support rotatif peut comprendre un séparateur comportant les logements, le séparateur étant en un matériau flexible, par exemple en élastomère, et un réceptacle recevant le séparateur. Les bandelettes de test peuvent facilement être montées dans le séparateur, qui peut ensuite être reçu dans le réceptacle pour former le support rotatif. La construction du support rotatif est facilitée. Le réceptacle peut comprendre typiquement une portion annulaire et une portion cylindrique, radialement externe à la portion annulaire. Le séparateur peut être collé sur le réceptacle, et plus spécifiquement sur la portion cylindrique.

Les bandelettes et le séparateur sur lequel les bandelettes sont montées sont appelés un ruban de mesure. Le ruban de mesure peut être fabriqué indépendamment du réceptacle puis peut être monté sur ce dernier.

Le séparateur peut comprendre des trous débouchant dans les logements, et le réceptacle peut comprendre une portion cylindrique transparente (ou ayant des zones transparentes) au contact du séparateur, de sorte que le système d'analyse détecte un résultat d'analyse par transmission de lumière au travers la portion cylindrique et les trous du séparateur. La lumière peut traverser le support rotatif via les bandelettes de test. Les trous peuvent guider la lumière vers des zones d'intérêt des bandelettes de test.

Le support rotatif peut comporter un ou plusieurs marqueurs, le dispositif peut comporter un capteur configuré pour détecter les marqueurs. Ainsi, le dispositif peut contrôler avec précision la position angulaire du support rotatif.

Les marqueurs peuvent être des marqueurs optiques, par exemple des lignes ou des codes-barres. Le capteur peut alors réaliser une analyse optique pour établir la position du support rotatif. En outre, le capteur optique peut identifier un type de bandelette de test. Le capteur peut également être le même que celui du système d'analyse. Moyennant quoi, la complexité du dispositif est réduite, en réduisant le nombre de composants exploités lors de son fonctionnement.

Dans un exemple, les marqueurs peuvent être des trous du séparateur débouchant dans les logements. Le capteur peut être le même que celui du système d'analyse.

Dans un exemple, les marqueurs peuvent comprendre une came, par exemple une came montée sur le support rotatif, qui est configurée pour coopérer avec un suiveur, monté sur la station. La came et le suiveur permettent d'obtenir une information sur la position angulaire de la cartouche dans la station. La came comprend typiquement une succession ou alternance de hauts et de bas sur un pourtour du support rotatif (par exemple sur une périphérie extérieure, comme celle du réceptacle), les hauts et bas étant agencés de sorte que chaque logement (et donc chaque bandelette) soit radialement aligné avec un bas (respectivement un haut). Une discontinuité de la came, identifiable par le suiveur, à une position donnée permet aussi de définir un zéro angulaire.

L'injecteur peut comprendre un moyen de contrôle d'urine, le moyen de contrôle d'urine comprenant : une douille définissant une cavité interne au travers laquelle de l'urine peut circuler ; une première électrode conductrice et une deuxième électrode conductrice traversant une paroi externe de la douille pour déboucher dans la cavité de sorte à être sensible à l'urine reçue dans la cavité, la première électrode étant distante de la deuxième électrode pour délimiter un volume de référence de la cavité. Un volume d'urine peut alors être mesuré avec précision. La répétabilité de l'injection d'urine sur une bandelette de test est améliorée.

Le moyen de contrôle d'urine peut comprendre une troisième électrode conductrice, disposée entre une extrémité de la douille et la première électrode conductrice pour mesurer un débit d'urine circulant dans la cavité. La mesure du débit d'urine peut permettre d'obtenir le temps d'activation d'une pompe permettant l'injection du volume contrôlé d'urine sur la bandelette de test. Les besoins de calibration de la pompe sont réduits.

L'injecteur peut être configuré pour injecter entre 1 microlitres et 20 microlitres d'urine sur une bandelette de test, par exemple entre 2 microlitres et 4 microlitres. Alors, l'injecteur injecte suffisamment d'urine sur une bandelette de test pour réaliser une analyse concluante.

Le système d'analyse peut comprendre : au moins une source de lumière (par exemple au moins une diode électroluminescente LED), disposée d'une part du support rotatif, l'au moins une source de lumière émettant de la lumière en direction du support rotatif, et, un capteur disposé d'autre part du support rotatif, le capteur faisant face à l'au moins une source de lumière de sorte à détecter la lumière traversant le support rotatif. Le système d'analyse est alors adapté pour réaliser une analyse par transmission de lumière au travers du support rotatif.

La station peut comprendre un moteur configuré pour entrainer en rotation le support rotatif.

Un boitier peut renfermer l'ensemble de test, le boitier étant configuré pour être entièrement positionné à l'intérieur de toilettes, en particulier à l'intérieur d'une cuvette de toilettes, contre une paroi intérieure de ladite cuvette. Le boitier protège alors l'ensemble de test de l'extérieur. Le dispositif est dépourvu de pièces mobiles à l'extérieur du boitier. Alors, le dispositif d'analyse d'urine est discret. Le boitier est en outre dépourvu de points de stagnation où de l'urine pourrait stagner. Le dispositif d'analyse d'urine est hygiénique. Le boitier est en outre dépourvu de pièce mobile extérieure au boitier, et donc dépourvu de joint tournant, problématique dans l'environnement considéré.

Le support rotatif peut être agencé de manière amovible dans le boitier (ou plus généralement dans la station). Ainsi, un utilisateur peut retirer le support rotatif pour le remplacer, par exemple pour recharger le dispositif en bandelettes de test ou changer le type de bandelettes de test.

Le boitier peut présenter une face avant destinée à recevoir un jet d'urine directement d'un utilisateur urinant en position assise sur les toilettes, une face arrière opposée à la face avant, et un orifice de collecte, disposé sur la face avant ou sur la face arrière. L'orifice de collecte peut également être situé au niveau d'une frontière entre la face avant et la face arrière. Ainsi, avantageusement, le dispositif d'analyse d'urine peut collecter de l'urine par l'orifice de collecte directement par ruissèlement le boitier. Un utilisateur n'a pas besoin de de se préoccuper de la position du boitier lorsqu'il urine dans les toilettes.

Le boitier peut comprendre un orifice de vidange configuré pour évacuer l'urine. Alors, un excès d'urine collecté peut être purgé du dispositif d'analyse d'urine collectée précédemment. Une collecte ultérieure d'urine n'est alors pas contaminée par une collecte précédente. Plusieurs collectes d'urine consécutives peuvent être réalisées.

Le boitier peut avoir une forme générale de galet circulaire. Ainsi, la forme du boitier est définie par des surfaces courbées. De l'urine peut ruisseler sur tout le boitier sans décrocher ou former de bulles d'air.

Le dispositif d'analyse d'urine peut comporter un capteur de présence d'urine, au voisinage de l'orifice de collecte, le capteur étant configuré pour détecter la présence d'urine, par exemple le capteur étant un capteur de température. Ainsi, le dispositif d'analyse d'urine peut déclencher une analyse lorsque de l'urine est détecté sur le boitier.

Le capteur de présence d'urine peut être un capteur de température. Le capteur de température permet de distinguer entre de l'urine et de l'eau étant détectée sur le boitier. En outre, le capteur de température peut détecter une période de fécondité. Alors, le capteur de température peut à la fois détecter la présence d'urine et réaliser une analyse. Le nombre de composants du dispositif d'analyse d'urine est réduit.

Le dispositif d'analyse d'urine peut comporter un module de communication, par exemple de communication sans-fil, avec un appareil déporté et/ou un serveur et/ou un smartphone. Ainsi, le dispositif d'analyse d'urine peut être commandé pour déclencher une analyse. Le dispositif d'analyse d'urine peut analyser et transmettre un ou plusieurs résultats d'analyses.

L'appareil déporté peut comprendre un bouton. Alors, une analyse peut être déclenchée lorsqu'un utilisateur appui sur le bouton.

Le bouton peut être pourvu d'un capteur biométrique. Alors, l'utilisateur peut être identifié et l'analyse ne s'enclencher que si un utilisateur est identifié. Une analyse peut être adaptée à l'utilisateur identifié.

Dans un mode de réalisation, il est aussi proposé un ruban de mesure pour cartouche de dispositif d'analyse d'urine. Ce ruban peut faire typiquement partie de la cartouche (aussi appelé support rotatif) décrite précédemment. Le ruban peut s'étendre selon une direction longitudinale et comprendre une pluralité de bandelettes disposées parallèlement les unes aux autres. Les bandelettes ont typiquement chacune une dimension maximale inférieure à 30 mm, voire 20mm, voire encore 15mm. Elles peuvent être disposées perpendiculairement à la direction longitudinale, de sorte que le ruban peut comprendre un nombre élevé de bandelettes tout en étant enroulable.

Les caractéristiques exposées dans les paragraphes suivants peuvent, optionnellement, être mises en oeuvre. Elles peuvent être mises en oeuvre indépendamment les unes des autres ou en combinaison les unes avec les autres.

Les bandelettes peuvent avoir une forme rectangulaire ayant chacune une largeur comprise entre 0,5 et 3 mm et une longueur comprise entre 10 et 15 mm.

Le ruban peut comprendre un séparateur, le séparateur comportant des logements pour recevoir les bandelettes. Le séparateur peut être en matière souple, comme de l'élastomère.

Le ruban peut comprendre au moins 50 bandelettes, voire au moins 100 bandelettes.

Le ruban peut être souple de façon à être enroulable selon un cercle ou un arc de cercle.

Le séparateur comprend des trous débouchant dans les logements.

Chaque logement peut être fermé par un opercule, par exemple un opercule transparent. L'opercule peut être continu pour faciliter le procédé d'assemblage.

Le ruban peut avoir une première face comprenant les logements et une deuxième face, opposée à la première face. La deuxième face peut comprendre au moins une saillie ou un renfoncement, configuré pour s'engager avec un renfoncement ou une saillie complémentaire du réceptacle de la cartouche.

Chaque logement peut comprendre deux parois latérales perpendiculaires à la direction longitudinale. De la sorte, les deux parois sont parallèles entre elles lorsque le ruban de mesure est à plat mais se rapprochent progressivement l'une de l'autre en s'inclinant vers l'intérieur du logement lorsque le ruban s'enroule (autrement dit lorsque l'axe longitudinal du ruban s'arrange en cercle et que les logements sont radialement internes et non pas radialement externes). Les bandelettes sont alors coincées dans leur logement par les parois inclinées.

Selon un autre aspect, il est proposé une méthode d'analyse d'urine exploitant le dispositif d'analyse d'urine, comprenant :
- positionner une bandelette de test devant l'injecteur par rotation du support rotatif ;
- injecter un volume contrôlé d'urine sur la bandelette de test ;
- positionner une bandelette de test devant le système d'analyse par rotation du support rotatif ;
- analyser la bandelette de test pour établir un résultat d'analyse, par exemple par analyse optique.

Les caractéristiques exposées dans les paragraphes suivants peuvent, optionnellement, être mises en oeuvre. Elles peuvent être mises en oeuvre indépendamment les unes des autres ou en combinaison les unes avec les autres.

La méthode peut comprendre mesurer un volume de référence, le volume de référence comprenant le volume contrôlé d'urine à injecter sur la bandelette de test. Le volume de référence peut également comprendre un volume de pré-injection. Le volume de pré-injection peut permettre d'expulser de l'air présent dans l'injecteur qui pourrait impacter le volume d'urine réellement injecté sur la bandelette de test.

Analyser la bandelette de test peut comprendre une étape de transmission de la lumière au travers le support rotatif. On réalise une analyse par transmission de lumière.

Injecter le volume contrôlé d'urine peut s'effectuer en deux temps. On prend alors en compte le temps d'absorption et de migration d'urine sur la bandelette de test.

La méthode peut être déclenchée par une étape préalable d'interaction avec un utilisateur. La méthode peut être déclenchée par l'appuie sur un bouton. La méthode peut être lancée par détection d'un utilisateur à proximité des toilettes. La méthode peut également être lancée lors de la détection d'urine sur le dispositif d'analyse d'urine.

Un utilisateur peut sélectionner une analyse. Par exemple, un utilisateur peut choisir le type d'analyse qu'il souhaite réaliser.

Le résultat d'analyse peut être transmis à un smartphone d'un utilisateur et/ou un serveur distant.

### Brève description des dessins

D'autres caractéristiques, détails et avantages apparaîtront à la lecture de la description détaillée ci-après, et à l'analyse des dessins annexés, sur lesquels :
[Fig. 1] représente schématiquement une vue en coupe de toilettes équipées d'un dispositif d'analyse d'urine au sens de l'invention.
[Fig. 2] représente schématiquement un détail de la Figure 1.
[Fig. 3] illustre une vue en perspective d'un premier boitier, selon un premier mode de réalisation, pouvant être mis en oeuvre dans le dispositif d'analyse d'urine de la figure 1.
[Fig. 4] illustre une vue en perspective éclatée du premier boitier de la figure 3 et d'un ensemble de test pouvant être mis en oeuvre dans le dispositif d'analyse d'urine de la figure 1.
[Fig. 5] illustre une vue en perspective partiellement éclatée d'un deuxième boitier, selon un deuxième mode de réalisation, pouvant être mis en oeuvre dans le dispositif d'analyse d'urine de la figure 1.
[Fig. 6] illustre un détail d'un troisième boitier, selon un troisième mode de réalisation, pouvant être mis en oeuvre dans le dispositif d'analyse d'urine de la figure 1.
[Fig. 7] illustre une autre vue en perspective du premier boitier de la figure 3.
[Fig. 8] illustre une vue en perspective de l'ensemble de test visible à la figure 4, d'un autre point de vue du côté opposé.
[Fig. 9] illustre une vue éclatée d'un premier exemple de sous-ensemble pouvant être mis en oeuvre dans l'ensemble de test.
[Fig. 10] illustre une vue en coupe transversale d'un détail du sous ensemble de la figure 9.
[Fig. 11] illustre une vue éclatée d'un deuxième exemple de sous-ensemble pouvant être mis en oeuvre dans l'ensemble de test.
[Fig. 12] illustre une vue en perspective d'un troisième exemple de sous-ensemble pouvant être mis en oeuvre dans l'ensemble de test.
[Fig. 13] illustre une vue en perspective d'un autre ensemble pouvant être mis en oeuvre dans l'ensemble de test.
[Fig. 14] illustre une bandelette de test pouvant être mise en oeuvre dans le dispositif d'analyse d'urine.
[Fig. 15] illustre un schéma block fonctionnel de l'ensemble de test de la figure 4.
[Fig. 16] illustre un organigramme d'un premier procédé d'utilisation du dispositif d'analyse d'urine.
[Fig. 17] illustre un organigramme d'un deuxième procédé d'utilisation du dispositif d'analyse d'urine.
[Fig. 18] illustre un quatrième exemple de boitier pouvant être mis en oeuvre dans le dispositif d'analyse d'urine monté dans des toilettes.
[Fig. 19] illustre une vue éclatée du quatrième exemple de boitier de la figure 18.
[Fig. 20] illustre une vue du dessus de l'intérieur du boitier de la figure 18.
[Fig. 21] illustre une vue en perspective d'un support rotatif comprenant une came, selon un mode de réalisation.
[Fig. 22] décomposée en deux figures FIG. 22a et FIG. 22b, illustre une vue du dessus d'un mécanisme de blocage/comptage, selon un mode de réalisation,
[Fig. 23] illustre une vue en perspective d'un mécanisme de blocage/comptage, selon un mode de réalisation.
[Fig. 24] illustre une vue en perspective d'un quatrième exemple de sous-ensemble pouvant être mis en oeuvre dans l'ensemble de test, focalisée sur un support rotatif.
[Fig. 25] illustre une vue partielle d'une première face d'un ruban de mesure, selon un mode de réalisation,
[Fig. 26] illustre une vue partielle d'une deuxième face d'un ruban de mesure, selon un mode de réalisation,
[Fig. 27] illustre une vue complète en perspective d'un ruban de mesure, avec un grossissement, selon un mode de réalisation.
[Fig. 28] illustre une vue zoomée du support rotatif, selon un mode de réalisation.
[Fig. 29] illustre une vue de côté d'un moyen de contrôle d'urine, selon un mode de réalisation.
[Fig. 30] illustre une vue en coupe d'un système d'analyse, selon un mode de réalisation
[Fig. 31] illustre une vue de dessus de composants électroniques pouvant commander l'ensemble de test, selon un mode de réalisation.

### Description des modes de réalisation

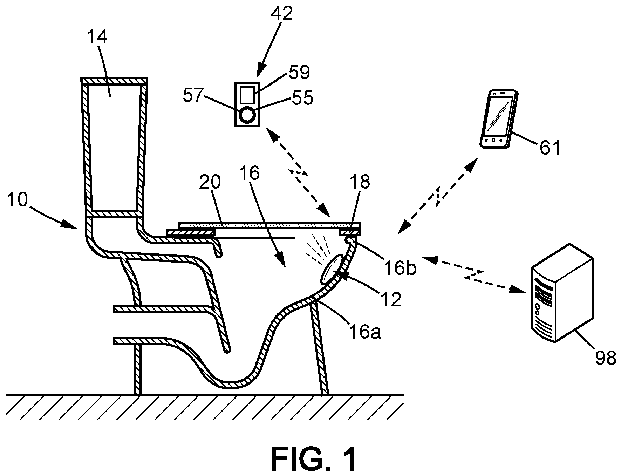

La figure 1 illustre des toilettes **10** équipées d'un dispositif d'analyse d'urine **12.** De manière connue, les toilettes comportent un réservoir d'eau **14,** une cuvette **16,** un siège **18** et un couvercle **20.** Le dispositif d'analyse d'urine est agencé sur une paroi interne **16a** de la cuvette **16** des toilettes. Avantageusement, le dispositif d'analyse d'urine **12** est entièrement reçu dans la cuvette des toilettes. En effet, le dispositif d'analyse d'urine est discret.

Ici, le dispositif d'analyse d'urine **12** est positionné sur la trajectoire d'un jet d'urine secrété par un utilisateur. Le dispositif d'analyse d'urine **12** reçoit un jet d'urine lorsqu'un utilisateur urine en position assise dans les toilettes. La position du dispositif d'analyse d'urine est alors adaptée pour tout type d'utilisateur, de sexe masculin ou féminin, quel que soit son âge. L'utilisateur peut alors uriner dans les toilettes sans se préoccuper de la position du dispositif d'analyse d'urine.

Ici, le dispositif d'analyse d'urine **12** est également positionné sur la trajectoire d'une chasse d'eau provenant du réservoir **14.** Ainsi, le dispositif d'analyse d'urine peut être rincé lors de l'actionnement de la chasse d'eau. Le dispositif d'analyse d'urine est hygiénique.

Le dispositif d'analyse d'urine **12** comprend un boitier **22** renfermant un ensemble de test **24.** L'ensemble de test **24** est destiné à analyser l'urine étant reçu dans le dispositif d'analyse d'urine **12.**

Le boitier **22** est agencé dans la cuvette **16** des toilettes de manière amovible. Le dispositif d'analyse peut alors être retiré ou repositionné dans les toilettes. Le dispositif d'analyse d'urine est discret. En outre, le dispositif d'analyse d'urine peut être retiré pour recharger une batterie **94** ou un support rotatif **44** de l'ensemble de test **24.**

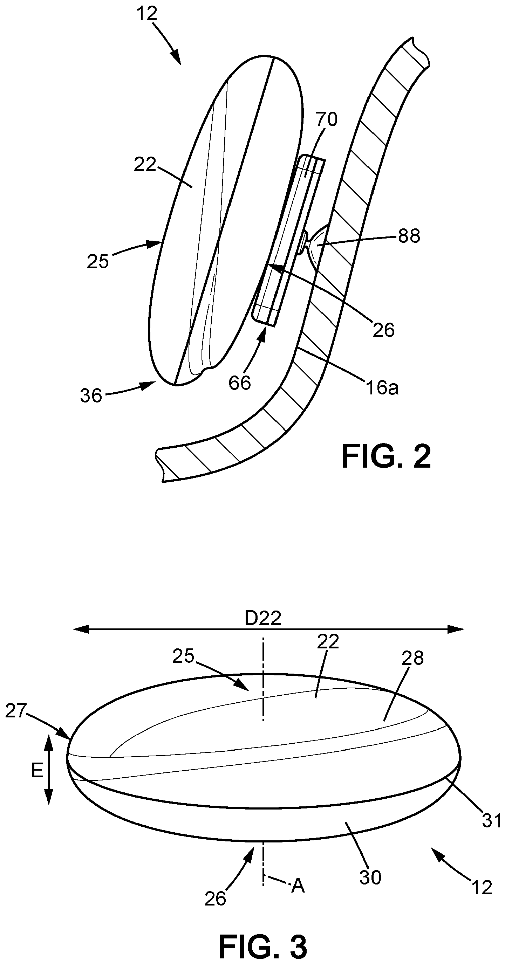

Dans l'exemple illustré à la figure 2, le boitier **22** est agencé sur la paroi 16a des toilettes. Le boitier est positionné par un élément de fixation **66.** L'élément de fixation **66** comporte une ventouse **88** destinée à coopérer avec la paroi **16a** des toilettes et des aimants **70.** Les aimants sont destinés à coopérer avec des aimants **23** (ou des pièces en matériau ferromagnétique) agencés à l'intérieur du boitier. Cette configuration permet de facilement retirer ou repositionner le boitier dans les toilettes.

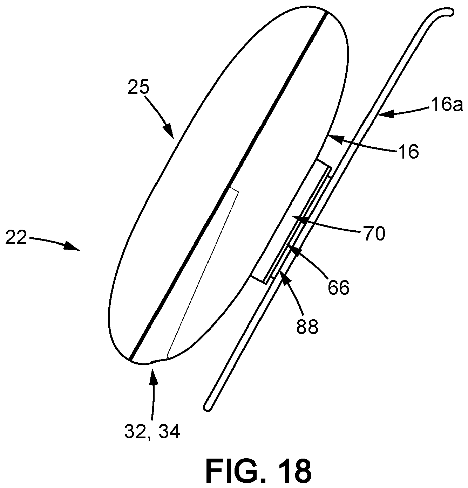

Dans l'exemple illustré à la figure 18, l'élément de fixation 66 est en outre de forme circulaire. L'élément de fixation 66 se loge dans un logement complémentaire prévu sur le boitier 22. Le logement est prévu au niveau de connecteurs de charge de la batterie 94 du dispositif d'analyse d'urine 12. Ainsi, les connecteurs de charge peuvent être protégés de l'eau des toilettes 10.

Des aides mécaniques, notamment des indentations, peuvent être prévus sur l'élément de fixation 66. Les aides mécaniques peuvent faciliter le positionnement du boitier 22 lors de son insertion dans les toilettes 10. La bonne position du boitier 22 dans les toilettes 10 assure le bon fonctionnement du dispositif d'analyse d'urine 12.

L'élément de fixation **66** peut également comprendre une liaison rotule. La liaison rotule permet d'orienter le boitier **22** afin d'augmenter la probabilité d'entrer en contact avec de l'urine étant reçu dans la cuvette **16** des toilettes.

En variante, l'élément de fixation **66** peut être un crochet monté sur un rebord **16b** de la cuvette des toilettes.

### Caractéristiques générales du boitier

Le boitier **22** a une forme extérieure de galet circulaire. En d'autres termes, le boitier a une forme de sphéroïde aplatie. Un axe **A** est l'axe médian du boitier. Le boitier comporte une face avant **25** et une face arrière **26,** sensiblement normales à l'axe A. Ainsi, une collecte d'urine peut se faire directement depuis les faces 25, 26 du boitier. Le boitier sert de collecteur d'urine.

La face avant **25** est orientée vers l'intérieur de la cuvette **16.** La face avant **25** est alors destinée à recevoir de l'urine lorsque l'utilisateur urine en position assise sur les toilettes. La face arrière **26** est orientée face à la paroi intérieure **16a** de la cuvette **16.** La face avant **25** et la face arrière **26** sont reliées par des bords courbés **27.** Alors, la surface extérieure du boitier **22,** constituée de la face avant **25,** la face arrière **26** et les bords courbés, est définie par des lignes courbes, formant un objet généralement convexe. Le boitier est dépourvu d'arêtes. De l'urine peut ruisseler sur l'ensemble de la surface extérieure du boitier sans décrocher du boitier ou former de bulles d'air, pouvant compromettre une analyse de l'urine.

La surface extérieure du boitier 22 peut en outre être de couleur blanche ou claire. La couleur de la surface extérieure peut être similaire à celle des toilettes, augmentant la discrétion du dispositif.

Dans un mode de réalisation, le boitier 22 a un diamètre **D22,** mesuré dans la direction normale à l'axe **A,** compris entre 50 mm et 150 mm, par exemple voisin de 100 mm. Le boitier 22 a également une épaisseur **E,** mesuré dans la direction de l'axe A, comprise entre 15 mm et 50 mm, par exemple voisine de 30 mm. Ainsi, le boitier est suffisamment compact pour être entièrement reçu dans la cuvette des toilettes. Le dispositif d'analyse d'urine est discret. En outre, le boitier est suffisamment étendu pour systématiquement entrer en contact avec de l'urine étant reçu dans la cuvette. L'utilisateur peut alors uriner dans les toilettes sans se soucier du dispositif d'analyse d'urine, ou à défaut viser sommairement.

La surface extérieure du boitier est lisse. Ainsi, le jet d'urine entrant en contact avec le boitier s'accroche et s'étale sur les surfaces extérieures du boitier. Dans un mode de réalisation, le boitier est d'un matériau hydrophile. Par exemple, le boitier peut être en un parmi : une céramique, un polyamide (PA), un silicone ou un polymère hydrophile. La surface extérieure du boitier peut également être traitée par un traitement de surface hydrophile, par exemple acuWet^{®} de la société Aculon, un polymère hydrophile, ou Pebax^{®} de la société Arkema.

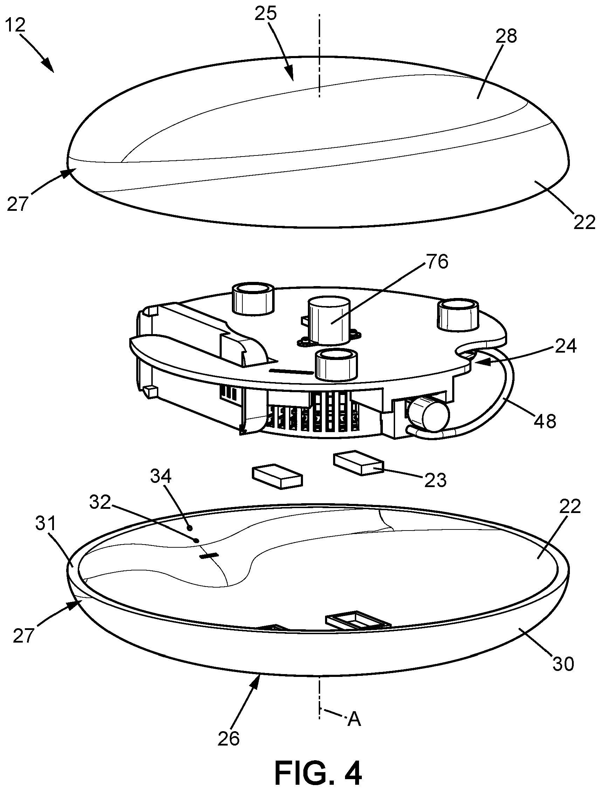

Comme plus visible sur la figure 4, selon une réalisation particulière, le boitier 22 est formé comme un assemblage de deux demi-coques et est ici constitué d'une coque avant **28** et d'une coque arrière **30.** La coque avant et la coque arrière forment une jointure **31** du boitier, dans un plan normal à l'axe **A.** L'assemblage du dispositif d'analyse d'urine est facilité lorsque le boitier est constitué de la coque avant et de la coque arrière.

La coque avant **28** et la coque arrière **30** sont assemblées pour maintenir la surface extérieure du boitier définie par des lignes courbes. Alors, la jointure 31 entre la coque avant et la coque arrière permet un ruissellement d'urine entre la face avant et la face arrière. L'impact de la jointure sur l'écoulement d'urine sur le boitier est minimisé.

La coque avant **28** et la coque arrière **30** peuvent être assemblées par vissage, collage, clipsage, par aimantation, ou soudure ultrason. Bien entendu d'autres moyens de fixation peuvent être mis en œuvre pour assembler la coque avant et la coque arrière.

Par exemple, la coque avant **28** et la coque arrière **30** sont assemblées par vissage. Alors, une portion interne de la coque avant comporte un filetage. Le filetage de la coque avant est destiné à coopérer avec un taraudage de la coque arrière. Le boitier peut ainsi facilement être démonté pour accéder à l'ensemble de test **24** à l'intérieur du boitier.

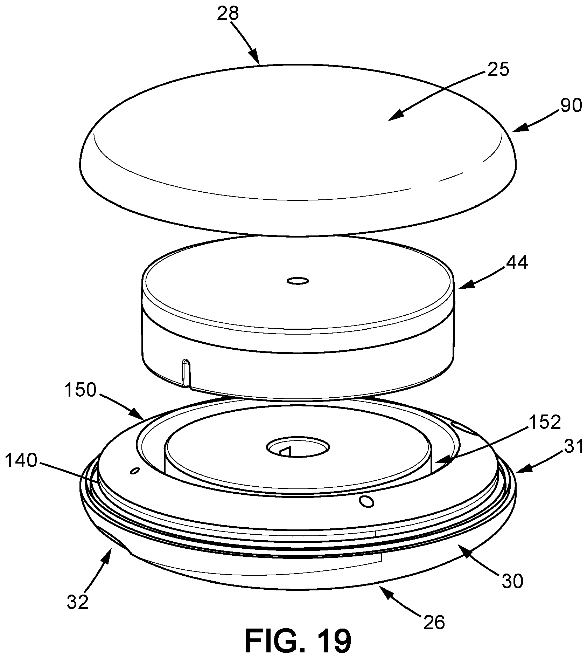

Dans un autre exemple de la figure 19, une portion interne de la coque arrière 30 comporte un filetage 140 destiné à coopérer avec un taraudage de la coque avant 28. On a un assemblage des deux coques 28, 30 par vissage. En alternative, l'assemblage des deux coques 28, 30 peut être un système à baïonnette.

Un joint d'étanchéité peut être présent au niveau de la jointure **31** entre la coque avant et la coque arrière. Ainsi, le boîtier est étanche. L'intérieur du boitier est imperméable à l'urine, à l'eau provenant du réservoir d'eau **14** ou de la cuvette **16,** et tout autre type de contaminant. Seuls des orifices de collecte et de vidange relient l'extérieur et l'intérieur du boitier, comme décrit plus en détail plus loin.

### Autres fonctions supportées par le dispositif

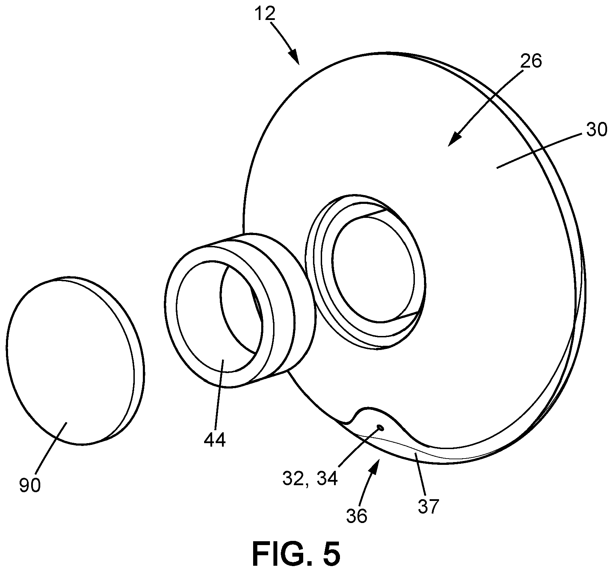

Dans l'exemple illustré à la figure 5, un capot amovible **90** est agencé sur la coque arrière. Le capot amovible permet de faciliter l'accès à l'ensemble de test **24,** en particulier à un support rotatif **44** de l'ensemble de test. Le capot amovible **90** permet notamment de recharger le support rotatif de l'ensemble de test.

Le capot amovible **90** est ici fixé sur la coque arrière **30** par clipsage, vissage ou par un mécanisme de baïonnette. Bien entendu, d'autres moyens de fixation peuvent être mis en oeuvre pour fixer le capot amovible **90** sur la coque arrière. Alternativement, dans un autre exemple, le capot amovible **90** pourrait être fixé sur la coque avant **28.**

Le capot amovible **90** est agencé de manière étanche. Par exemple, une jointure entre le capot amovible **90** et la coque arrière **30** peut comporter un joint d'étanchéité. Ainsi, l'intérieur du boitier **22** reste imperméable à l'urine, à l'eau provenant du réservoir d'eau **14** ou de la cuvette **16,** et tout autre type de contaminant.

Dans un autre exemple, illustré à la figure 18, le capot amovible 90 est formé par la coque avant 28 du boitier 22. Le capot amovible 90 peut alors être retiré par dévissage de la coque avant 28 par rapport à la coque arrière 30. Le boitier 22 comporte moins de jointures pouvant être salies et/ou infiltrées par l'eau des toilettes.

Le boitier **22** présente un orifice de collecte **32.** L'orifice de collecte **32** peut recevoir l'urine ruisselant par gravité sur la surface extérieure du boitier. Une collecte d'urine est réalisée directement depuis les faces **25, 26** du boitier.

L'orifice de collecte **32** est situé sur une extrémité inférieure **36** du boitier **22.** L'extrémité inférieure **36** est orientée vers le fond de la cuvette **16** lorsque le boitier **22** est positionné dans la cuvette **16** des toilettes. Cette position correspond à une position normale d'utilisation. Cette position permet une collecte de l'urine ruisselant par gravité sur la majorité de la surface extérieure du boitier.

En l'espèce, une distance **D** séparant l'orifice de collecte **32** d'une bordure inférieure 22a du boitier est inférieure à 40 mm, par exemple inférieure à 20mm. Selon une réalisation particulière, l'orifice de collecte 32 est agencé à quelques millimètres au-dessus de la bordure inférieure du boîtier. En variante, l'orifice de collecte peut être sur la bordure inférieure 22a.

L'orifice de collecte **32** est une ouverture circulaire, de diamètre par exemple compris entre 0,3 mm et 2 mm. Le diamètre de l'orifice de collecte peut être choisi afin de maximiser le volume d'urine collecté de la surface extérieure du boitier.

Le boitier présente un orifice de vidange **34.** L'orifice de vidange **34** permet de purger le dispositif d'analyse d'urine **12** d'un excès d'urine.

L'orifice de vidange **34** peut être distinct de l'orifice de collecte **32.** Alors, l'orifice de vidange est également situé sur l'extrémité inférieure du boitier, au voisinage de l'orifice de collecte. L'orifice de vidange est également une ouverture circulaire. L'orifice de vidange a un diamètre compris entre 0,3 mm et 2 mm. Dans la position normale d'utilisation, l'orifice de vidange se trouve en dessous de l'orifice de collecte.

L'orifice de vidange 34 peut également être éloigné de l'orifice de collecte 32. La position de l'orifice de vidange 34 peut être choisie pour faciliter l'accès à l'orifice de vidange par l'ensemble de test 24.

En variante, comme illustré à la figure 5, l'orifice de vidange **34** peut être le même que l'orifice de collecte **32.** Un unique orifice permet de limiter le nombre d'ouvertures vers l'intérieure du boitier. Alors, le risque d'introduction de contaminants ou d'éléments susceptibles d'obstruer l'ensemble de test est réduit.

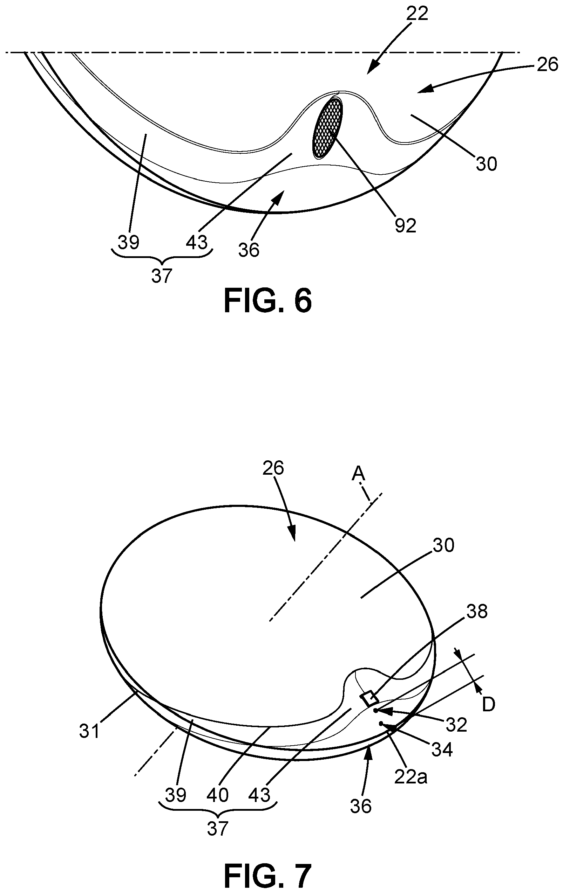

Comme visible sur la figure 6, l'orifice de collecte **32** et l'orifice de vidange **34** peuvent être surmontés par un filtre **92** en maille métallique. Le filtre recouvre les orifices 32, 34. Le filtre en maille est par exemple de forme oblongue recouvrant les orifices 32,34. L'ouverture moyenne de maille du filtre est par exemple de 20 microns. Le filtre permet d'empêcher l'introduction de contaminants ou d'éléments susceptibles d'obstruer l'ensemble de test **24,** et de filtrer l'urine reçue dans l'orifice de collecte. Par ailleurs, le filtre pourrait être nettoyé grâce à un flux d'air généré par une pompe à air.

Dans les exemples illustrés, l'orifice de collecte et l'orifice de vidange sont situés sur la face arrière du boitier. Alors, l'orifice de collecte et l'orifice de vidange font face à la paroi intérieure **16a** de la cuvette lorsque le dispositif d'analyse d'urine est positionné dans les toilettes. Cette position permet de masquer l'orifice de collecte et l'orifice de vidange par la face avant du boitier. Aussi, cette position permet d'empêcher l'introduction de contaminants ou d'éléments susceptibles d'obstruer l'ensemble de test.

L'orifice de collecte et l'orifice de vidange sont situés dans un renfoncement 37. Le renfoncement **37** comporte deux rainures latérales **39** s'étendant depuis la jointure **31** du boitier vers une partie centrale **43** du renfoncement **37** comportant les orifices **32, 34.** La profondeur des rainures latérales **39,** soit la distance depuis la face arrière **26** vers l'intérieur du boitier selon la direction de l'axe **A,** croit depuis la jointure jusqu'à la partie centrale **43.** Ainsi, le renfoncement **37** forme une amenée d'urine depuis la face avant **25** jusqu'à l'orifice de collecte. Avantageusement, le renfoncement permet de récolter de l'urine ruisselant sur la face avant et l'acheminer vers l'orifice de collecte. Ainsi, le volume d'urine atteignant l'orifice de collecte depuis la face avant du boitier est suffisant pour les besoins de l'analyse.

Une bordure **40** délimitant le renfoncement est arrondie. Autrement dit, la bordure est définie par des surfaces courbées. La bordure est dépourvue d'arête. De l'urine au contact de la face arrière peut ruisseler vers l'orifice de collecte sans décrocher du boitier ou former des bulles d'air. Ainsi, le volume d'urine atteignant l'orifice de collecte depuis la face arrière du boitier est augmenté.

Le boitier 22 ne se limite pas aux seuls modes de réalisation décrits ci-avant en regard des figures, mais est, au contraire, susceptible de nombreuses variantes accessibles à l'homme de l'art.

Le boitier peut notamment prendre toute forme géométrique définie par des lignes courbes. Le boitier peut notamment avoir une forme de losange ou de goutte inversée. Alors, le boitier comporte une pointe sur la partie inférieure afin de guider l'urine vers l'orifice de collecte.

L'orifice de collecte et l'orifice de vidange peuvent être sur la face avant du boitier. Ainsi, l'urine ruisselant sur la face avant atteint l'orifice de collecte de manière plus directe.

L'orifice de collecte et l'orifice de vidange peuvent être situés sur un relief positif, notamment une saillie, ou négatif, notamment une gouttière ou un renfoncement. De manière générale, le relief peut être de toute géométrie permettant de canaliser l'urine ruisselant sur le boitier et de l'acheminer vers l'orifice de collecte sans décrocher du boitier ou former des bulles d'air.

Dans un exemple de réalisation, l'orifice de collecte **32** est agencé sur la face avant **25,** alors que l'orifice de vidange **34** est situé sur la face arrière **26.**

### Ensemble de test, injecteur, système d'analyse

Par la suite, on décrit plus en détail un ensemble de test **24** exploitant des bandelettes colorimétriques. Les bandelettes colorimétriques sont ici également désignées « bandelettes de tests » **56.**

L'ensemble de test **24** est contrôlé par une unité électronique de commande **45.** L'unité électronique de commande 45 est à l'intérieur du boitier 22. L'unité électronique de commande **45** permet de contrôler les composants de l'ensemble de test **24** pour réaliser une analyse d'urine exploitant les bandelettes de test **56** et obtenir un ou plusieurs résultats d'analyses.

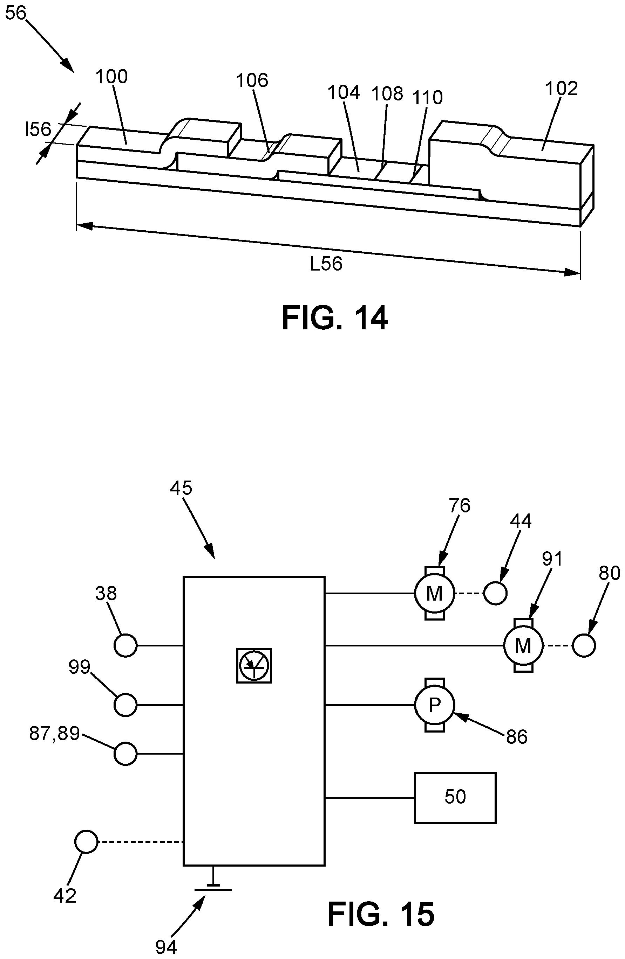

Comme visible sur la figure 14, les bandelettes de test **56** sont du type immunodosage à flux latéral ou vertical, en anglais « *latéral or vertical flow immunoassay* ». Alors, les bandelettes de test **56** comportent un tampon d'échantillonnage **100** et un tampon d'absorption **102.** Une membrane de nitrocellulose **104** s'étend entre le tampon d'échantillonnage **100** et le tampon d'absorption **102.** Alors, lorsqu'un échantillon d'urine est introduit sur le tampon d'échantillonnage **100,** il migre par capillarité jusqu'au tampon d'absorption **102** en passant au travers d'un tampon conjugué **106,** d'une ou plusieurs lignes de test **108,** et d'une ligne de contrôle **110.** Le tampon conjugué **106,** le ou les lignes de test **108** et la ligne de contrôle **110** contiennent des réactifs.

Le tampon conjugué **106** comporte notamment des anticorps de détection sensibles à des composés contenus dans l'urine. Si les composés sont présents lorsque l'échantillon d'urine traverse le tampon conjugué **106,** alors les anticorps se fixent aux composés pour former des marqueurs. Les marqueurs migrent vers une ligne de test **108.** La ligne de test comporte notamment des anticorps de test. Les anticorps de test se lient avec les marqueurs et les retiennent sur la ligne de test **108.** Alors, une ligne colorée se forme et la densité de la ligne varie en fonction de la concentration de marqueurs présents. L'échantillon restant migre vers une ligne de contrôle **110.** La ligne de contrôle comporte des anticorps de contrôle, permettant d'indiquer que l'échantillon a traversé la membrane de nitrocellulose **104.**

Par exemple, les bandelettes de test **56** peuvent être des bandelettes de type ELISA. Ce type de bandelette de test **56** permet une détection de l'hormone de grossesse hCG dans l'urine. Alors, l'anticorps de détection peut être « mouse monoclonal beta hCG », l'anticorps de test peut être « *goat poluclonal anti-mouse IgG* » et l'anticorps de contrôle peut être « *rabbit polyclonal anti-mouse igG ».*

En variante, les bandelettes de test **56** peuvent être des bandelettes colorimétriques classiques. Alors, chaque bandelette de test comporte au moins un tampon contenant un ou plusieurs réactifs sensibles à un ou plusieurs composés contenus dans l'échantillon d'urine. Par exemple, le ou les composés peuvent être : l'hormone LH, l'hormone HCG, les leucocytes/nitrites, les urobilinogène/bilirubine, les protéines, le pH, la gravité spécifique et/ou le glucose.

D'autres types de réactions peuvent utiliser réactifs ou composés conçus pour détecter la présence d'un analyte particulier (par exemple des polymères à empreinte moléculaire "MIP" ou "*Molecularly imprinted polymers*"), notamment un principe actif de médicament ou un métabolite de principe actif de médicament dans l'urine. Dans ce cas, le dispositif peut être utilisé pour contrôler l'observance d'un utilisateur pour un traitement médicamenteux, notamment vérifier qu'il prend bien son traitement ou l'alerter lorsqu'il a omis de le prendre.

Chaque bandelette de tests **56** est globalement rectangulaire. Une largeur **I56** de chaque bandelette de test **56** peut être comprise entre 0,5 mm et 3 mm, par exemple environ 1 mm. Une longueur **L56** de chaque bandelette de test peut être comprise entre 10 et 15 mm, par exemple 12 mm ou environ 12 mm. Alternativement, chaque bandelette de test peut avoir une forme quelconque, par exemple carrée ou circulaire. La forme et les dimensions des bandelettes de test permettent de stocker un nombre important de bandelettes de test dans le dispositif d'analyse d'urine **12** (au moins 50 bandelettes, voire au moins 100 bandelettes). En effet, il apparait possible de stocker jusqu'à 120 bandelettes de test, ce qui correspond à 4 mois d'analyses lorsqu'un utilisateur réalise une analyse par jour.

L'ensemble de test **24** exploitant les bandelettes de tests comprend spécifiquement un ou plusieurs supports rotatifs **44,** un injecteur 46, un moyen d'acheminement d'urine **48** et un système d'analyse **50.**

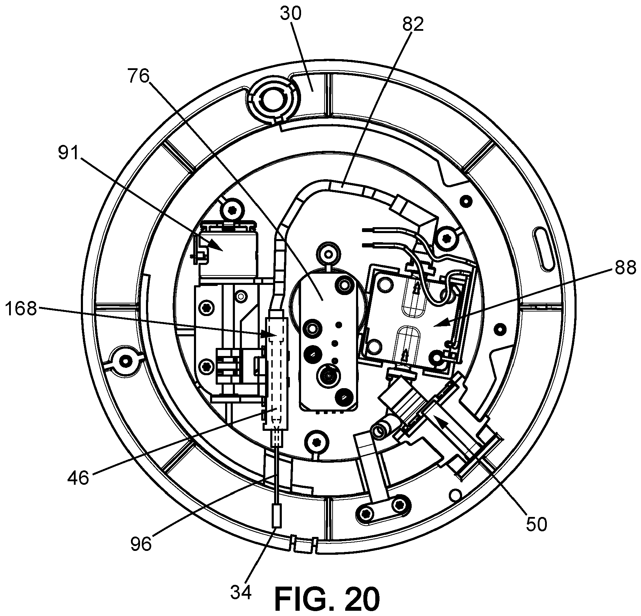

L'ensemble de test **24** est agencé sur une embase **68.** L'embase permet de positionner, voire de fixer, l'injecteur **46,** le ou les supports rotatif **44,** le moyen d'acheminement d'urine **48** et le système d'analyse **50** dans le boitier. En variante, comme dans l'exemple de la figure 20, l'injecteur 46, le moyen d'acheminement d'urine 48 et le système d'analyse 50 sont montés directement sur le boitier 22, en particulier sur la coque arrière 30 du boitier 22. L'embase 68 est ainsi formée par la coque arrière 30 du boitier 28.

Le dispositif d'analyse d'urine 12 comprend ainsi une station avec un ou plusieurs supports rotatifs 44. La station est donc définie comme le dispositif d'analyse d'urine 12 en excluant le ou les supports rotatifs 44. Comme déjà expliqué, le support rotatif 44 est amovible de la station, de sorte la station et support rotatif 44 peuvent être physiquement séparés (par exemple pour être fabriqués et/ou vendus indépendamment l'un de l'autre). Un utilisateur final ou un intermédiaire peut ensuite les assembler. On utilisera aussi indifféremment le terme de cartouche pour désigner le support rotatif.

Dans l'exemple de la figure 19, un couvercle 150 recouvre les composants de l'ensemble de test 24, à l'exception du support rotatif 44. Le couvercle 150 ferme la station. Le couvercle 150 comprend un logement 152 pour recevoir le support rotatif 44. Cette configuration permet de protéger les composants de l'ensemble de test 24 tout en permettant un accès au support rotatif 44. Dans ce cas, le capot amovible 90 peut être sur ou formé par la face avant du boiter pour permettre d'accéder au logement 152 et changer le support rotatif 44.

### Support rotatif

Les bandelettes de tests **56** sont stockées dans le support rotatif **44.** Dans un mode de réalisation, le support rotatif **44** est de forme cylindrique creuse s'étendant annulairement autour d'un axe qui est, lorsque le support rotatif 44 est monté dans la station, l'axe médian A du boitier **22** (par commodité de langage, on utilisera un seul axe A pour décrire les différents éléments), en pratique le support rotatif est généralement symétrique de révolution autour de l'axe **A.** Le support rotatif **44** permet de stocker un nombre important de bandelettes de tests **56** tout en étant suffisamment compact pour être agencé à l'intérieur du boitier.

Le support rotatif 44 tel qu'illustré sur les figures s'étend sur un tour complet et peut effectuer dans la station un tour complet. Toutefois, il peut être envisagé, pour des raisons d'encombrement ou pour permettre de libérer de l'espace pour d'autres composants, un support rotatif 44 qui s'étend sur une portion de tour (par exemple moins de 180° ou 90°) et qui n'effectue en rotation qu'une portion de tour (par exemple moins de 270°). Dans ce cas, le nombre de bandelettes 56 est typiquement moins élevé que pour le dispositif d'analyse d'urine illustré sur les figures.

Les bandelettes sont agencées selon un cercle ou une portion de cercle, par exemple à une extrémité radiale du support rotatif 44 pour maximiser leur nombre. Le positionnement en cercle assure que les bandelettes sont toutes à la même distance de l'axe de rotation et, de ce fait, de l'injecteur 46 ou du système d'analyse 50 (en particulier un capteur optique du système d'analyse 50, qui sera décrit plus tard). Cela assure aussi que le protocole de mesure pour chaque bandelette est identique. Comme illustré sur les figures, les bandelettes peuvent être disposées parallèlement entre elles, et plus spécifiquement, parallèlement à l'axe A.

De par sa forme et sa fonction, le support rotatif **44** s'apparente à un barillet.

En l'espèce, un diamètre extérieur **D44** du support rotatif **44** peut être compris entre 30 mm et 130 mm, de préférence environ 60 mm. Une hauteur **H44** du support rotatif, mesurée selon la direction de l'axe **A** peut être compris entre 12 mm et 40 mm, de préférence environ 14 mm. Un rapport entre le diamètre **D44** du support rotatif **44** et le diamètre **D22** du boitier **22** peut être supérieur ou égal à 0,3, de préférence supérieur ou égal à 0,5. On obtient ainsi une solution de très bonne compacité au regard du nombre important de bandelettes de tests disponibles.

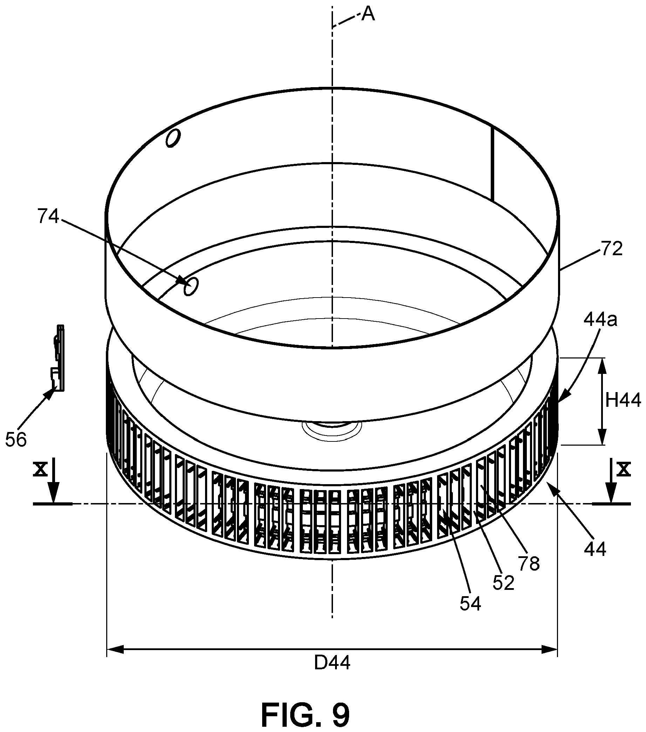

Dans un premier exemple, les bandelettes de test **56** sont reçues dans une paroi extérieure **44a** du support rotatif **44** (illustré notamment les figures 9 à 13). Moyennant quoi, le nombre de bandelettes de test **56** pouvant être stockées par le support rotatif est encore plus important.

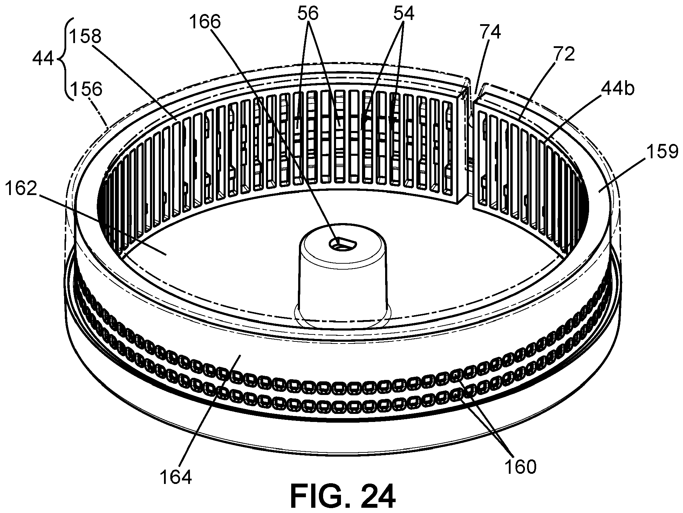

Alternativement, dans un exemple décrit en détail plus loin (en référence aux figures 24 à 28), les bandelettes de test peuvent être stockées dans une paroi intérieure du support rotatif. Cette disposition permet d'éviter que l'utilisateur ne touche les bandelettes lorsqu'il manipule le support rotatif. Cette configuration permet en outre une flexibilité sur le positionnement de l'injecteur et du système d'analyse par rapport au support rotatif.

Selon un autre exemple non illustré, le support rotatif **44** pourrait être une rondelle, d'axe confondu avec l'axe médian **A** du boitier. La rondelle s'étend alors radialement, selon un plan sensiblement normal à l'axe A médian du boitier. Les bandelettes de test peuvent alors être stockées sur une face de la rondelle, normale à l'axe A. Cette configuration permet notamment d'adapter le support rotatif à différentes formes de boitier. Alors, le support rotatif peut être implémenté dans divers analyseurs d'urine.

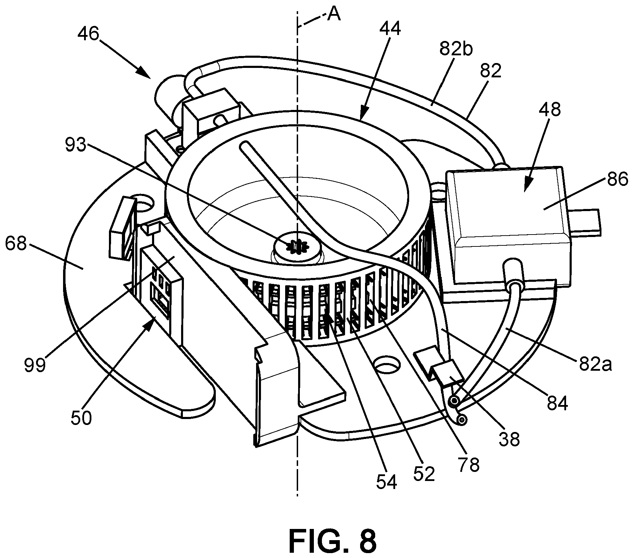

Tel qu'illustré (notamment en figures 8 à 10), la paroi extérieure **44a** du support rotatif présente une succession de rainures **52** délimités par des murets **78.** Les murets forment des logements **54** pour les bandelettes de test. Les murets permettent d'isoler un logement **54** de logements voisins selon la direction circonférentielle. Alors, les logements 54 peuvent séparer les bandelettes de test **56** à être utilisées lors d'analyses successives. Les bandelettes de tests usées sont séparées de bandelettes de test neuves.

Les rainures **52** s'étendent ici selon la direction de l'axe A, sensiblement sur toute la hauteur du support rotatif **44.** Alors, le support rotatif peut comporter entre 40 et 150 logements, de préférence entre 60 et 120. Ainsi, un nombre important bandelettes de test peut être stocké dans le support rotatif.

En variante, les rainures **52** pourraient s'étendre selon la direction radiale. Cette configuration apparait intéressante pour augmenter encore le nombre de logements du support rotatif **44,** et stocker davantage de bandelettes de test.

Chaque logement **54** peut recevoir une unique bandelette de test **56.** Toutes les bandelettes de test des logements peuvent être d'un même type. Par même type, on entend qu'elles sont sensibles aux mêmes composés contenus dans l'urine. Le support rotatif est alors adapté pour une analyse spécifique.

En variante, la bandelette de test **56** reçue dans le logement **54** peut être d'un type différent de la bandelette de test reçue dans le logement voisin. Ainsi, plusieurs types d'analyses, requérant différents types de bandelettes de test, peuvent être réalisées à partir du même support rotatif **44.**

En variante, chaque logement **54** peut contenir une pluralité de bandelettes de test de types différents. Ainsi, plusieurs types d'analyses peuvent être réalisées à partir d'un même logement.

Le support rotatif **44** comporte une ouverture **74.** L'ouverture permet le passage d'une aiguille **96** de l'injecteur **46** au travers du support rotatif, notamment pour réaliser une évacuation de l'urine contenue dans le dispositif d'analyse d'urine **12.**

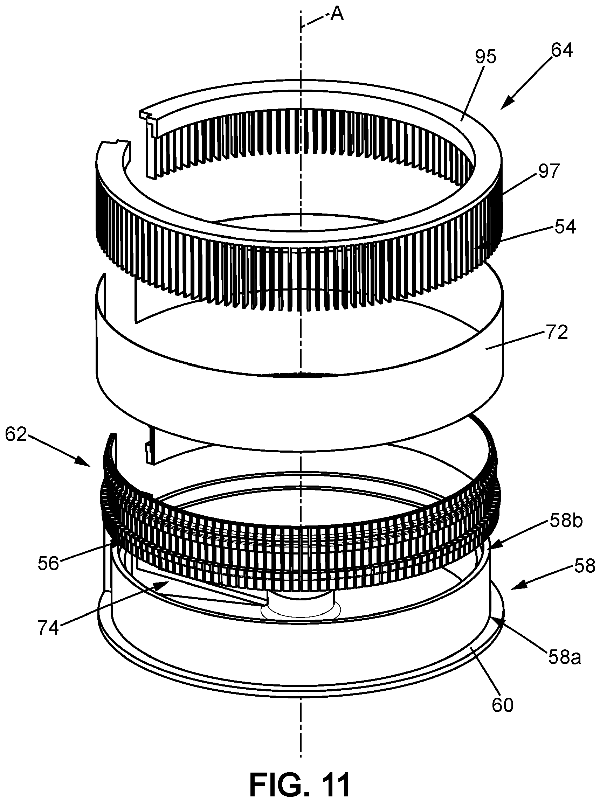

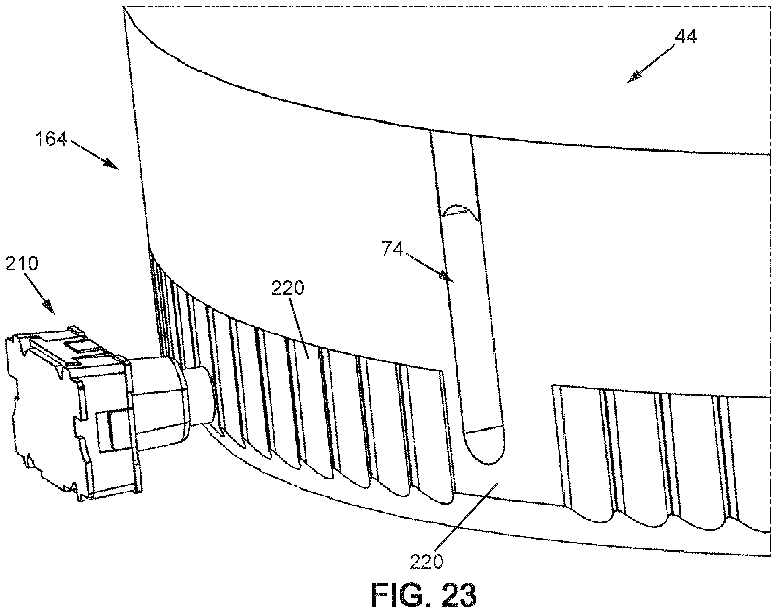

Dans l'exemple de la figure 9, l'ouverture **74** est une ouverture circulaire, s'étendant radialement au travers du support rotatif. Alternativement, l'ouverture 74 peut être une fente s'étendant sur toute la hauteur **H44** du support rotatif. L'ouverture peut également être oblongue, comme illustrée en figure 23. Alternativement encore, l'ouverture **74** peut être définie par un secteur angulaire, tel qu'illustré à la figure 11, 24 ou 28. Le secteur angulaire peut avoir la forme d'une encoche formée dans le support rotatif (voir figure 24 par exemple). La forme de l'ouverture permet de faciliter la fabrication du support rotatif, notamment lors d'une fabrication par moulage par injection.

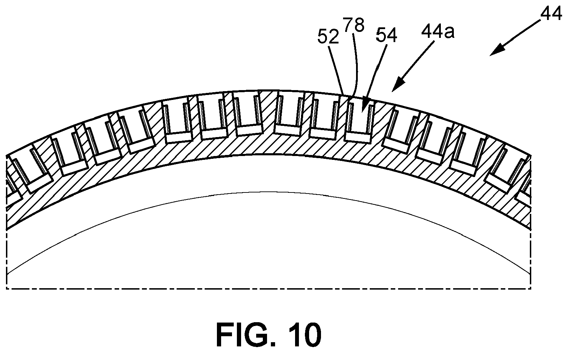

Dans l'exemple illustré à la figure 9, le support rotatif **44** est constitué d'une unique pièce. Comme plus visible sur la figure 10, les rainures **52** sont alors disposées par groupes de 3 dans un plan tangentiel à la paroi extérieure **44a** du support rotatif. Cette disposition limite le nombre d'inserts nécessaire à la fabrication du support rotatif lors d'une fabrication par moulage par injection.

Alternativement, dans l'exemple illustré à la figure 11, le support rotatif **44** est formé par deux pièces. Une première pièce **58,** dite 'armature' prend la forme d'un cylindre creux ou anneau s'étendant autour de l'axe **A,** en étant coaxial avec celui-ci. Une nervure annulaire **60,** s'étendant radialement vers l'extérieur, entoure une extrémité **58a** de la première pièce armature **58.** Alors, les bandelettes de test **56** peuvent être positionnées sur la première pièce armature **58.** Les bandelettes de test peuvent alors être reliées pour former un ruban **62.** Une deuxième pièce **64** comporte une bague **95.** La bague **95** est destinée à coopérer avec l'extrémité **58b** de la première pièce armature **58,** opposée à la première extrémité **58a** selon la direction de l'axe **A,** pour former le support rotatif.

La bague **95** comporte une succession de projections **97** s'étendant selon la direction de l'axe A, en position assemblée, jusqu'à la nervure annulaire **60** de la première pièce armature **58.** Les projections **97** séparent les bandelettes de test **56** du ruban **62.** Alors, les projections **97** forment les murets des logements **54** recevant les bandelettes de test. Cette configuration facilite la manipulation et l'assemblage des bandelettes de test dans le support rotatif.

### Opercule

Chaque logement **54** est recouvert et fermé par un opercule **72.** L'opercule permet d'isoler hermétiquement les bandelettes de test reçues dans un logement de l'environnement extérieur et de logements voisins. Alors, avant une analyse, les réactifs des bandelettes de test sont protégés d'une éventuelle contamination. En outre, après une analyse, l'opercule **72** peut contenir de l'urine introduite dans le logement **54.**

Comme notamment visible sur les figures 9 et 11, l'opercule **72** peut prendre la forme d'un film continu. Le film est collé sur la paroi extérieure **44a** du support rotatif pour recouvrir les logements **54.** Cette configuration facilite la mise en place de l'opercule **72** sur le support rotatif.

Une telle construction permet un assemblage simple et susceptible d'être automatisé, à partir de composants relativement simples. Le coût de revient d'un support rotatif équipé de bandelettes de test est ainsi avantageux.

Alternativement, chaque logement **54** peut être recouvert par un opercule **72** distinct. Cette configuration permet de limiter le risque de contamination de bandelettes de tests reçues dans deux logements voisins.

Alternativement encore, les bandelettes de test **56** peuvent être encapsulées individuellement. Cette configuration apparait particulièrement intéressante lorsque les bandelettes de test sont reliées pour former un ruban 62. En effet, le ruban **62** peut être assemblé dans le support rotatif sans nécessiter l'ajout d'un opercule **72** supplémentaire. L'assemblage du dispositif d'analyse d'urine **12** est facilité.

L'opercule **72** est ici en un matériau inerte. Par exemple, l'opercule peut être en silicone ou en acrylique. De préférence, l'opercule est de qualité médicale, pour éviter la contamination des bandelettes de tests avec des produits indésirables contenus dans l'opercule. Alors, les réactifs des bandelettes de test sont conservés intacts avant une analyse.

En outre, l'opercule **72** est transparent, un taux de transparence étant de préférence supérieur à 99%. Alors, une analyse par colorimétrie peut être réalisée sur une bandelette de test au travers l'opercule.

### Moteur

Le support rotatif **44** est assemblé sur l'arbre d'un moteur **76** (figure 4) pour être entrainé en rotation autour de l'axe **A.** Le support rotatif peut alors être sélectivement positionné pour aligner une bandelette de test face à l'injecteur **46** ou face à au système d'analyse **50.** Ainsi, l'usage du support rotatif permet d'avoir un équipage mobile simple avec un seul axe de rotation. En outre, cette configuration réduit les contraintes liées au positionnement et l'agencement de l'injecteur **46** et du système d'analyse **50** dans le dispositif d'analyse **12.**

Alternativement, le moteur 76 pourrait être non aligné à l'axe A du support rotatif 44. Comme montré à la figure 20, le moteur 76 peut par exemple être décalé de l'axe A, avec un réducteur à engrenages reliant le rotor du moteur 76 au moyeu support rotatif 44. L'engrenage peut permettre une meilleure précision de la position angulaire du support rotatif 44.

Il n'est pas exclu d'avoir le moteur 76 et son arbre de sortie agencés radialement à l'extérieur du support rotatif 44. Par exemple, la paroi extérieure du support rotatif 44 peut présenter une succession de dents coopérant avec des dents liées à l'arbre du moteur 76. En variante, le support rotatif 44 pourrait être fixé sur un disque présentant la succession de dents.

Le moteur **76** peut entrainer le support rotatif 44 selon la direction horaire ou antihoraire. Le support rotatif peut alors rapidement atteindre la position désirée, suivant la trajectoire la plus courte. Ainsi, on réduit encore les contraintes liées au positionnement et l'agencement de l'injecteur **46** et du système d'analyse **50** dans le dispositif d'analyse **12.**

Le moteur **76** est par exemple un moteur pas-à-pas. Alors, le moteur pas-à-pas permet une indexation contrôlée du support rotatif. Alternativement, le moteur peut être un moteur à courant continu.

### Marqueurs

La position angulaire du support rotatif **44** peut être contrôlée par la détection d'un ou plusieurs marqueur(s) sur le support rotatif. Le ou les marqueurs permettent de contrôler avec précision la position du support rotatif dans le dispositif d'analyse d'urine. L'unité électronique de commande 45 est couplé à un capteur configuré pour détecter la présence dans une position particulière du ou des marqueurs.

Le support rotatif peut être inséré dans le dispositif d'analyse d'urine de manière aléatoire, et le positionnement du support rotatif peut avantageusement être réalisé automatiquement, après une étape d'initialisation consistant en une rotation « à l'aveugle » jusqu'à un repérage d'au moins un susdit marqueur.

Il peut être prévu un marqueur ponctuel sur le support rotatif, ce marqueur ponctuel faisant office de repère angulaire 'zéro'. A partir de la connaissance de cette position angulaire de référence dite 'zéro'. Ensuite le pilotage du moteur pas-à-pas mémorise le nombre de pas effectués dans chaque direction permet à l'unité électronique de commande de suivre continuellement la position angulaire courante du support rotatif. Ce processus est effectué en boucle ouverte, mais il peut être prévu un recalage éventuel à chaque fois que le marqueur 'zéro' se retrouve en face du capteur.

Dans un exemple, le ou les marqueurs peuvent par exemple être des marqueurs magnétiques.

Dans un autre exemple, le ou les marqueurs peuvent par exemple être des marqueurs optiques. Les marqueurs optiques peuvent être des lignes ou des codes-barres sur le support rotatif. Les marqueurs optiques sont destinés à coopérer avec un capteur optique **99.** Avantageusement, l'usage d'un capteur optique peut également permettre d'identifier le type de bandelettes de test dans le support rotatif. En outre, le même capteur optique **99** peut réaliser l'analyse par colorimétrie sur les bandelettes de test. Cette configuration permet de réduire la complexité et les coûts liés à la fabrication du dispositif d'analyse d'urine, en limitant le nombre de composants exploités lors de son fonctionnement.

### Mécanisme de blocage et/ou de comptage par came

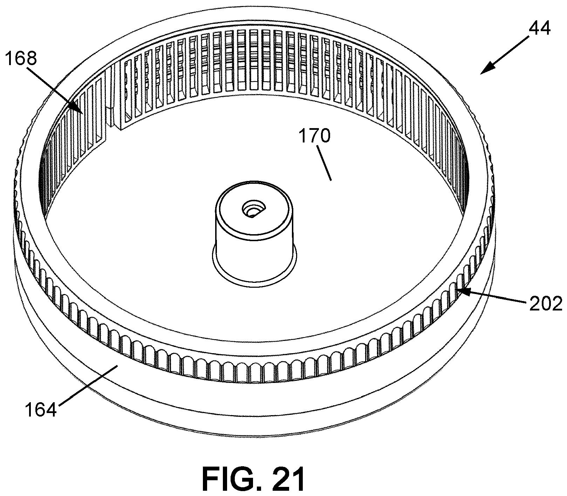

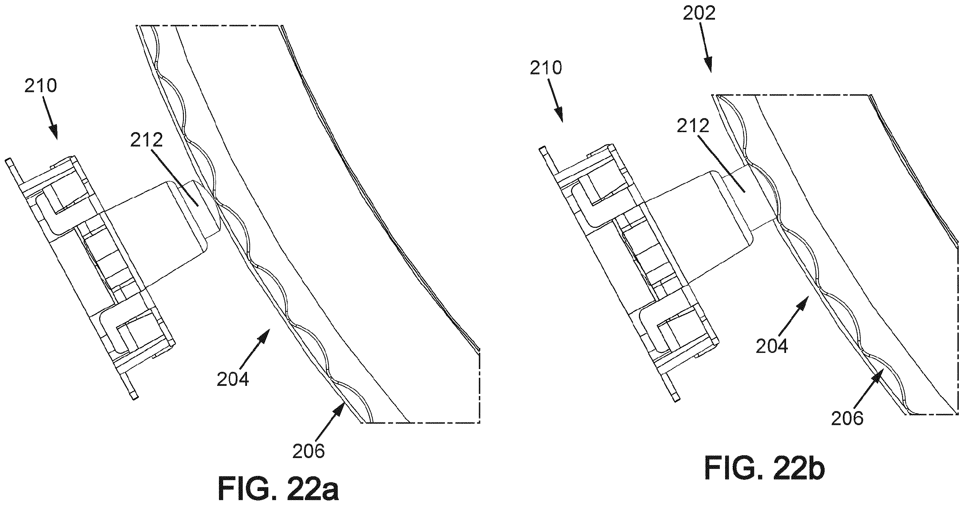

Dans un mode de réalisation illustré sur les figures 21 à 23, un mécanisme de blocage et/ou de comptage est incorporé au dispositif d'analyse d'urine 12. Le support rotatif 44 peut comprendre, sur une périphérie externe (typiquement une périphérie externe d'une portion annulaire 162 ou d'une portion cylindrique 164, qui seront décrites par la suite), une came 202 présentant une alternance de hauts 204 et de bas 206. En d'autres termes, le rayon du support rotatif 44 varie angulairement au niveau de la came 202. En regard de cette came 202 se trouve un suiveur 210, fixe (le suiveur fait partie de la station), configuré pour identifier les hauts 204 et les bas 206 de la came 202. Le suiveur peut typiquement comprendre une tige 212 mobile en translation et poussée par défaut en direction de la came 202 par un ressort (non visible sur les figures), de sorte que la tige 212 se déplace en fonction des hauts 204 et des bas 206 de la came 202, comme respectivement illustré par les figures 22a, 22b. Dans un exemple, le suiveur a une sortie binaire, correspondant à un haut ou à un bas. Il est ainsi possible de compter le nombre de hauts 204 et de bas 206 franchis lorsque le support rotatif 44 tourne dans la station. Chaque bas 206 (alternativement chaque haut) est associé à (par exemple radialement aligné avec) une bandelette de test 56 du support rotatif 44 (ou à un logement 54 recevant la bandelette), de sorte que le mécanisme de comptage permet de savoir exactement combien de bandelettes ont défilé devant le suiveur 210 et, partant, à l'aide d'un repère (soit le zéro, soit la dernière position connue), permet de savoir quelle bandelette est au niveau de l'injecteur 46 et/ou du capteur optique 99.

La came 202 est donc un mode de réalisation des marqueurs précités.

Le suiveur 210 peut être un microrupteur, par exemple un microrupteur à sortie binaire.

Un tel mécanisme de comptage permet notamment de s'affranchir de l'utilisation d'un moteur pas à pas et facilite l'utilisation d'un moteur conventionnel, de type à courant continu. De plus, la commande du moteur 76 peut être faite à l'aide des données obtenues par le suiveur 210, ce qui garantit que toute imprécision liée au moteur ne dérèglera pas le dispositif d'analyse d'urine 12.

Dans une variante, la came peut présenter un motif monodirectionnel, de sorte que la came et le suiveur fonctionne comme un cliquet n'autorisant la rotation que dans un seul sens.

Comme représenté en figure 23, au niveau de l'ouverture 74, la came 202 peut avoir une portion 220 plate ou présentant un motif différent des hauts et des bas du reste de la came 202, de sorte que le suiveur 210 peut identifier le repère 'zéro' angulaire. Par exemple, pour une vitesse de rotation donnée du moteur 76, le suiveur 210 va maintenir la même sortie pendant une durée supérieure à un seuil ou supérieure à la durée de passage d'un haut ou d'un bas.

Dans ce mode de réalisation, le capteur optique 99 n'est pas utilisé pour compter ou identifier la position angulaire du support rotatif 44. Pour simplifier l'implémentation de la came 202, le suiveur 210 peut être angulairement positionné au même endroit que l'optique 99, c'est-à-dire que lorsque le suiveur est en regard d'un bas (ou un haut) de la came, le capteur optique 99 est aussi en regard de la bandelette et du logement qui sont associé à ce bas (ou ce haut) de la came.

La came 220 et le suiveur 210 permettent aussi, en complément ou alternativement au mécanisme de comptage, de générer une butée qui s'oppose à la rotation du support rotatif 44. En particulier, lors d'une inversion du sens de rotation du moteur 76, la chaine d'engrenages peut subir un *backlash* qui nuit à la précision du système. En bloquant la rotation du support rotatif 44, il est possible d'assurer que le *backlash* soit compensé avant tout mouvement du support rotatif 44. En outre, comme le moteur 76 peut être commandé en utilisant les données du suiveur 210, le moteur 76 peut patiner, ralentir, accélérer ou sauter une ou plusieurs dents d'engrenage sans générer de décalage accidentel entre les bandelettes de test 56. Le mécanisme de blocage peut aussi être activé pendant une injection d'urine sur la bandelette de test 56, voire pendant la phase d'analyse de la bandelette. Pour augmenter la force de blocage, le suiveur 210 peut alors comprendre un mode bloqué, qui empêche la translation de la tige 212 et de ce fait participe à immobiliser le support rotatif 44. Dans ce mode de réalisation, on privilégie un alignement radial d'un bas avec une bandelette.

Alternativement, la came 202 et le suiveur 210 peuvent être inversés (suiveur monté sur le support rotatif et came montée sur la station). Toutefois, cette solution est moins commode à implémenter et moins économique.

D'autres types de suiveurs peuvent être montés dans le dispositif d'analyse d'urine (avec une roue qui roule sur la came, avec une lame en flexion, etc.). De la même façon, au lieu d'un microrupteur binaire, le suiveur peut avoir davantage de sorties.

### Montage du support rotatif

Le support rotatif **44** est monté dans le dispositif d'analyse d'urine de manière amovible. Alors, le support rotatif peut être retiré du boitier. Le support rotatif peut être remplacé, notamment pour recharger l'ensemble de test 24 en bandelettes de tests ou changer le type de bandelettes de tests. Alors, le dispositif d'analyse d'urine est modulable et polyvalent.

Dans l'exemple de la figure 8, le support rotatif **44** présente un manchon femelle à cannelure **93** apte à être assemblé sur un pignon mâle denté de l'arbre du moteur **76.** Le support rotatif peut alors être facilement retiré et inséré du boitier par translation le long de l'axe **A,** sans compromettre la rotation du support rotatif.

Comme illustré sur la figure 5, le boitier **22,** comportant un capot amovible **90,** est particulièrement adapté pour retirer le support rotatif **44** du boitier. En effet, le support rotatif est facilement accessible. En outre, le capot amovible permet de protéger le reste de l'ensemble de test **24** lors du retrait du support rotatif.

Comme décrit plus haut, dans un exemple, le capot amovible **90** est agencé sur la face avant 25 du boitier. Dans un autre exemple, le capot amovible **90** est agencé sur la face arrière 26 du boitier. Dans encore un autre exemple, le capot amovible 90 est formée par la face avant 25 du boitier.

Comme illustré à la figure 5, il est prévu une rainure **90a** sur la face externe du capot amovible. Cette rainure **90a** permet de faire tourner capot amovible **90** en y insérant une pièce de monnaie par exemple. La profondeur de la rainure peut être de 1 mm à 1,5 mm. Sa longueur est voisine du diamètre **D44** et sa largeur peut être de 2 mm à 2,5 mm.

Alternativement, comme illustré à la figure 19, le capot amovible 90 est retiré par dévissage de la coque avant 28 par rapport à la coque arrière 30.

### Variante double barillet

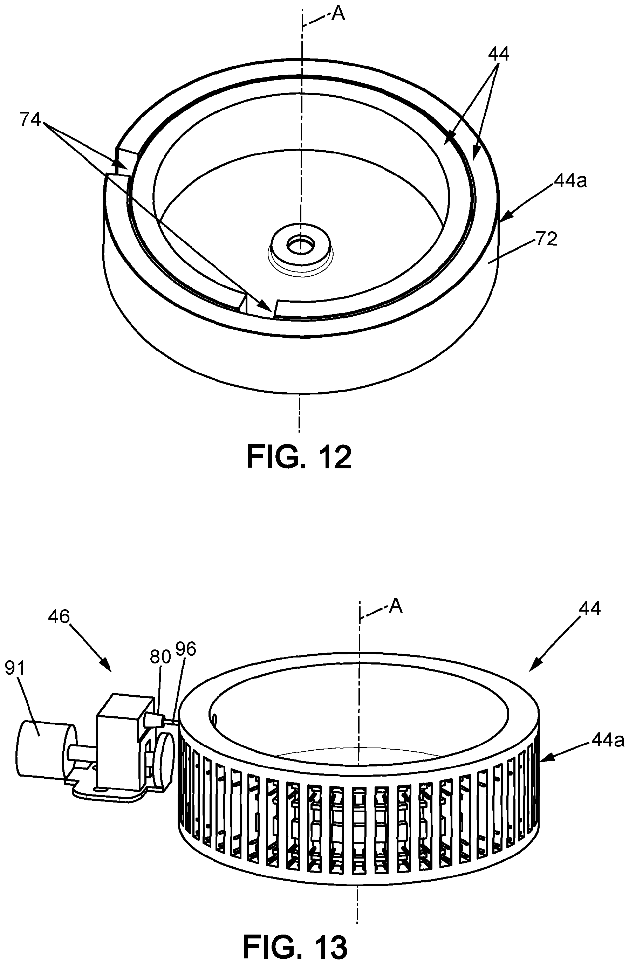

Dans l'exemple illustré à la figure 12, l'ensemble de test **24** comporte deux supports rotatifs tels que décrits ci-avant, à savoir un premier support rotatif **44** à l'extérieur et un deuxième **442** agencé à l'intérieur du premier. Les deux supports rotatifs **44** sont coaxiaux selon l'axe A. Deux supports rotatifs permettent de stocker davantage de bandelettes de test dans le boitier. Alors, chaque support rotatif **44,442** comporte une ouverture **74** sous forme de fente. Les fentes permettent en outre le passage de l'aiguille **96** pour accéder aux logements **54** de l'un ou l'autre des supports rotatifs **44,442.**

Chaque support rotatif **44,442** est piloté en position angulaire par son propre moteur, deux moteurs étant alors agencés en configuration coaxiale.

### Variante support rotatif

Les figures 24 à 28 illustrent un autre exemple de support rotatif 44. Ici, les bandelettes 56 sont stockées dans une paroi intérieure 44b du support rotatif 44. Le support rotatif 44 peut être reçu dans le logement 152 du couvercle 150 recouvrant les autres composants de l'ensemble de test 24.

Le support rotatif 44 comprend un réceptacle 156 et un séparateur 158.

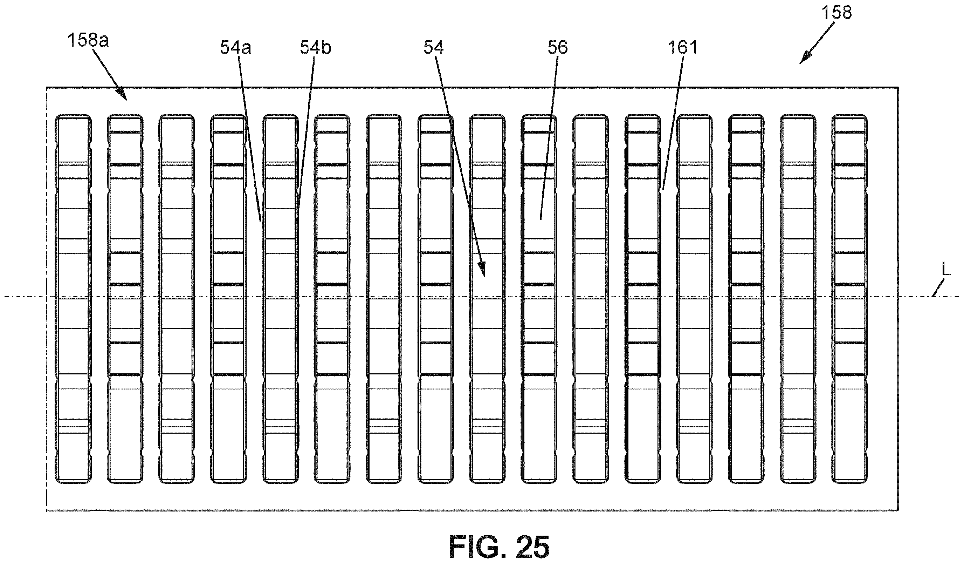

Le séparateur 158 est une pièce flexible, notamment en élastomère en forme de bande ou ruban destinée à être enroulée dans le réceptacle 156. Comme illustré en figures 25, 26 et 27, le ruban (et aussi le séparateur 158) s'étend le long d'une direction longitudinale L. Le séparateur 158 comprend une première face 158a comprenant les logements 54 recevant les bandelettes de test 56. La première face peut être recouverte par l'opercule 72 (visible schématiquement sur la figure 24). Les logements 54 sont scellés. L'insertion des bandelettes de test 56 dans les logements 54 peut alors se faire sur une surface plane. Le montage est simplifié.

Chaque logement 54 peut comprendre deux parois latérales parallèles 54a, 54b (i.e. les parois perpendiculaires à la direction longitudinale L), afin de faciliter l'insertion des bandelettes 56. En outre, une fois le séparateur 158 enroulé et monté dans le réceptacle 156, la courbure en cercle incline les parois latérales 58a, 58b qui se referment progressivement en direction de la surface du séparateur (c'est-à-dire en direction de l'opercule 72). Cela permet de coincer les bandelettes 56 à l'intérieur de leur logement 54 d'une façon efficace et simple. Alternativement ou complémentairement, chaque logement peut comporter, sur au moins une paroi latérale 54a, 54b, une nervure 161 (typiquement intégrale avec le séparateur 158) qui nécessite de légèrement contraindre la bandelette 56 pour pouvoir l'insérer dans son logement 54. Cette nervure 161 concourt à maintenir la bandelette à l'intérieur de son logement. Sur la figure 25, chaque logement 54 comprend quatre paires de nervures 161, chaque paire comprenant deux nervures se faisant face.

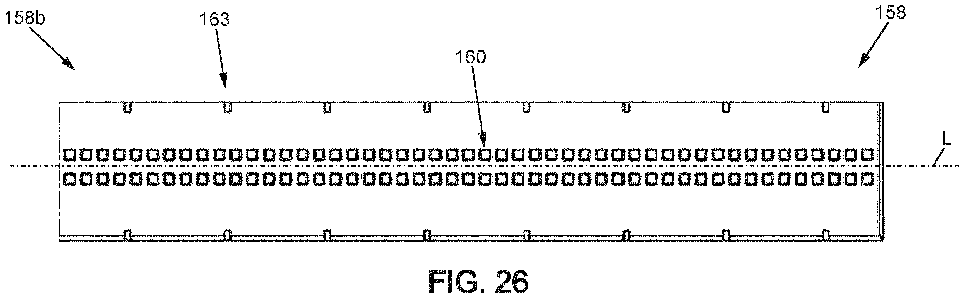

Comme illustré en figure 26, le séparateur 158 comprend également une deuxième face 158b, opposée à la première face 158a. La deuxième face 158b présente des trous traversants 160. Les trous débouchent dans les logements 54 recevant les bandelettes de test 56. En particulier, chaque logement 54 comprend au moins un trou 160. Les trous 160 sont alignés avec des zones d'intérêts de chaque bandelette de test 56, par exemple les lignes de tests 108. Les trous 160 permettent de guider la lumière vers les bandelettes 56 pour les analyser par colorimétrie. Cela permet de positionner la bandelette entre la source de lumière 176, 178 d'un côté et le capteur optique 99 de l'autre côté. La lumière peut être guidée à travers les trous 160 vers les zones d'intérêt de la bandelette de test 56. On peut ainsi prévoir deux trous 160 par logement 54, pour analyser deux zones d'intérêt d'une bandelette de test 56.

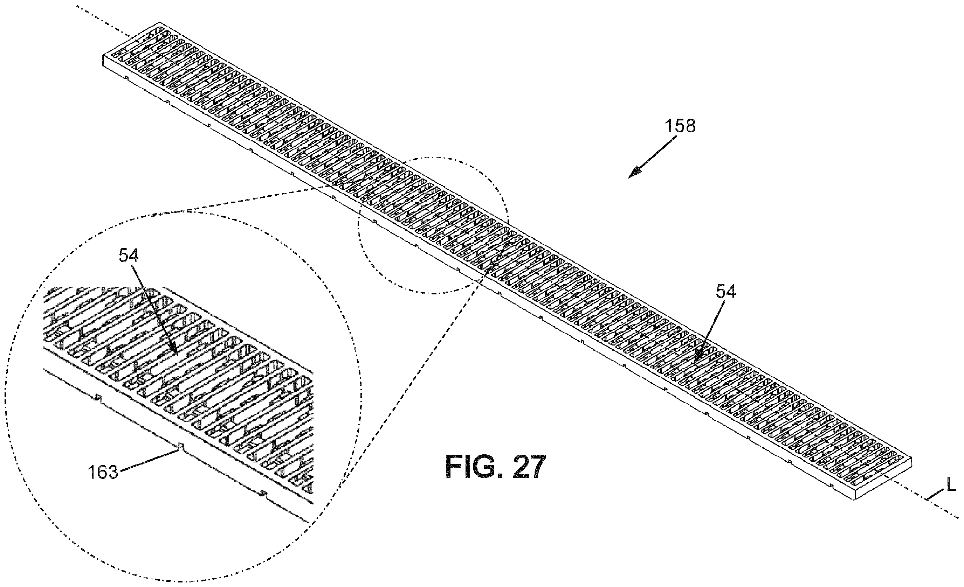

Le séparateur 158 peut comprendre, par exemple sur la deuxième face 158b, au moins un renfoncement 163 ou une saillie (renfoncement visible sur les figures 26 et 27). Complémentairement, le réceptacle 156 comprend une saillie ou un renfoncement (respectivement) pour coopérer avec le renfoncement ou la saillie du séparateur (respectivement). Cela permet notamment de s'assurer que les logements 54 (et donc les bandelettes 56) sont bien alignés radialement avec les bas (ou les hauts) de la came 202. Une pluralité de renfoncements/saillies 163 peut être prévue entre le séparateur 158 et le réceptacle 156 pour assurer que chaque logement 54 soit bien radialement aligné avec un bas ou un haut de la came 206. En effet, le séparateur 158, étant en matériau souple, peut présenter des irrégularités de fabrication, que le ou les couples renfoncement/saillies permettent de compenser, en maintenant étirées ou compressées certaines portions du séparateur 158 une fois ce dernier mis en place.

En complément ou alternative au renfoncement/saillie 163, le séparateur 158 peut être collé sur le réceptacle 156. Une colle transparente aux fréquences utilisées pour l'analyse optique est typiquement utilisée.

Comme illustré en figure 24, le réceptacle 156 comprend une portion annulaire 162 et une portion cylindrique 164, radialement externe à la portion annulaire 162

La portion cylindrique 164 est transparente, ou bien comprend des zones transparentes. La portion cylindrique 164 est au contact du séparateur 158, en particulier la deuxième face du séparateur 158. Lors d'une analyse par colorimétrie, la lumière peut traverser la portion cylindrique 164 pour analyser les bandelettes de test 56.

La portion cylindrique 164 peut notamment être en polycarbonate. En effet, le polycarbonate présente des bonnes propriétés de transmission de la lumière, tout en restant relativement peu coûteux et compatible avec un procédé de moulage par injection.

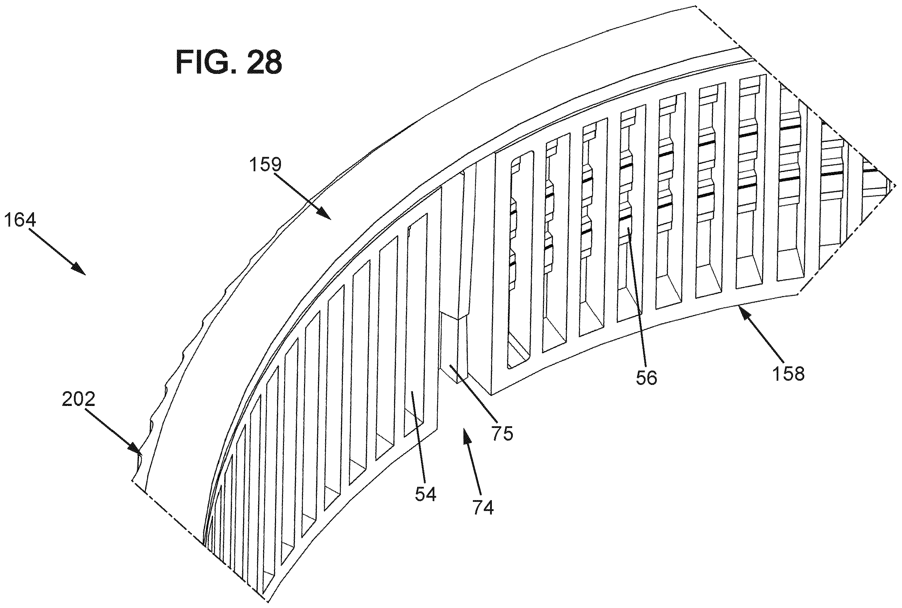

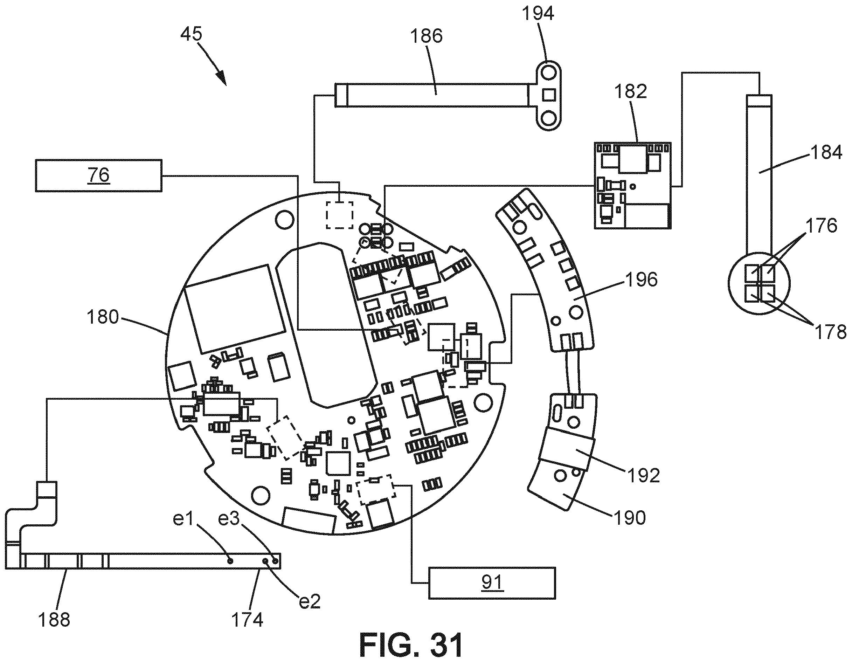

La portion annulaire 162 du réceptacle 156 forme un socle pour porter le séparateur 158. La portion cylindrique 164 reçoit le séparateur 158. Le séparateur 158 est bloqué en translation selon la direction A par un contact avec la portion annulaire 162. De l'autre côté, comme illustré en figure 31, un rebord annulaire 159 s'étendant depuis une extrémité de la portion cylindrique 164 et radialement vers l'intérieur permet de bloquer la translation selon l'autre sens de la direction A. Le rebord annulaire 159 s'étend par exemple sur une distance similaire à l'épaisseur au séparateur 158. Pour bloquer la rotation du séparateur 158 dans le réceptacle 156, outre la colle précédemment décrite et les renfoncements/saillies 163 décrits précédemment, ou en alternative à celle-ci et/ou ceux-ci, au moins une butée 75 (voir figure 28) peut être montée sur le réceptacle 156 (typiquement sur la portion cylindrique 164). Par exemple, la butée 75 se trouve sur la portion cylindrique 164, au niveau de l'ouverture 74, pour que l'extrémité du séparateur 158 vienne au contact de la butée 75. Une autre butée peut être prévue symétriquement de l'autre côté de l'ouverture, contre laquelle l'autre extrémité du séparateur peut venir buter.

La portion annulaire 162 comprend en outre un manchon femelle 166 de fixation du moteur 76. Le manchon femelle 166 forme le moyeu destiné à recevoir en son centre l'arbre du moteur 76 ou d'un réducteur interposé. Ici, le manchon femelle 166 est aligné à l'axe A du support rotatif 44. Toutefois, dans l'exemple où le moteur 76 est radialement externe au support rotatif 44, la portion annulaire 162 pourrait être dépourvue du manchon femelle 166. La portion annulaire 162 pourrait être montée par tout type de liaison pivotante par rapport au boitier 22. La portion annulaire 162, lorsqu'elle n'est pas pourvue d'un trou traversant au niveau de l'axe A peut s'apparenter à un disque.

Par ailleurs, les marqueurs optiques peuvent ici être l'ouverture 74 du support rotatif 44 et chacun des trous 160 du séparateur 158. En effet, l'ouverture 74 du support rotatif 44 peut faire office de repère angulaire 'zéro' et chaque trou 160 peut ensuite permettre d'obtenir la position angulaire du support rotatif 44 par rapport au repère angulaire 'zéro'. Injecteur

L'injecteur **46** comporte une seringue automatisée **80,** sur laquelle l'aiguille **96** est orientée vers les logements du ou des supports rotatifs. L'injecteur **46** est destiné à percer l'opercule **72** et injecter un échantillon d'urine sur une bandelette de test.

Dans l'exemple illustré à la figure 13, l'injecteur **46** est agencé à l'extérieur du support rotatif. La seringue automatisée **80** s'étend radialement en direction de l'axe **A.** Alors, l'aiguille **96** pointe en direction de la paroi extérieure **44a** du support rotatif. Alternativement, par exemple lorsque les bandelettes de test sont stockées dans une paroi interne du support rotatif, l'injecteur pourrait être agencé radialement à l'intérieur du support rotatif.

La seringue automatisée **80** injecte, par l'aiguille **96,** un volume contrôlé d'urine sur une bandelette de test, par exemple entre 2,5 microlitres et 3,5 microlitres. Ainsi, la seringue automatisée **80** injecte un volume suffisant d'urine sur une bandelette de test pour réaliser une analyse concluante sans risquer un débordement d'urine du logement.

La seringue automatisée 80 peut injecter un volume contrôlé d'urine sur la bandelette de test 56 en deux temps. Par exemple, la seringue automatisée 80 peut injecter deux fois entre 2,5 microlitres et 3,5 microlitres. Cette solution permet de prendre en compte un temps de réaction et de migration de l'urine sur la bandelette de test 56.

L'aiguille **96** présente une ouverture latérale. L'ouverture latérale permet le passage de l'échantillon d'urine depuis la seringue vers les logements. L'ouverture latérale permet d'éviter une obstruction du bout de l'aiguille lors de plusieurs perçages successifs.

L'injecteur **46** peut comprendre un premier capteur **89.** Le premier capteur est agencé en amont de la seringue automatisée **80.** Le premier capteur permet alors de détecter si de l'urine est reçue dans la seringue automatisée. L'injecteur peut en outre comprendre un deuxième capteur **87.** Le deuxième capteur **87** est agencé dans la seringue automatisée **80,** à proximité de l'aiguille **96.** Alors, le deuxième capteur peut vérifier que la seringue automatisée contient de l'urine pour réaliser une injection. Le ou les capteurs **89, 87** peuvent alors contrôler l'injection d'urine sur la bandelette de test. Alternativement, l'injecteur 46 peut comprendre un moyen de contrôle du volume d'urine 168, comme sera décrit plus en détail plus loin.

La seringue automatisée **80** est agencée sur un moteur linéaire **91.** Le moteur linéaire permet de déplacer radialement la seringue automatisée. Au repos, la seringue automatisée est éloignée du support rotatif pour permettre une rotation du support rotatif. En outre, la seringue automatisée peut être rapprochée du support rotatif pour atteindre une bandelette de test. La seringue automatisée **80** peut également traverser l'ouverture **74** du support rotatif, notamment pour évacuer un excès d'urine de la seringue automatisée.

En outre, dans l'exemple comportant deux supports rotatifs **44,442** la seringue automatisée **80** peut être déplacée radialement pour atteindre une bandelette de test de l'un ou l'autre des supports rotatifs **44.** Il est donc prévu une sur-course pour atteindre le deuxième support rotatif **442.**

### Pompe et canalisations

Selon un premier exemple, le moyen d'acheminement d'urine **48** comporte un canal de collecte **82,** un canal de purge **84** et une pompe **86.** Le canal de collecte relie l'orifice de collecte **32** à l'injecteur **46,** en particulier à la seringue automatisée **80.** Alors, de l'urine peut atteindre la seringue automatisée depuis l'orifice de collecte. Le canal de purge **84** relie l'injecteur, en particulier la seringue automatisée **80,** à l'orifice de vidange **34.** Le canal de purge permet alors l'évacuation de l'urine contenue dans le dispositif d'analyse d'urine.

Ici, le canal de purge **84** est agencé radialement intérieurement au support rotatif. La seringue automatisée **80** accède au canal de purge **84** au travers l'ouverture **74** du ou de chaque support rotatif. Alors, l'évacuation d'urine peut se faire par injection de la seringue automatisée dans le canal de purge. Le dispositif d'analyse d'urine peut être dépourvu de composants particulièrement dédiés à l'évacuation d'urine. La complexité du dispositif d'analyse d'urine est réduite.

La seringue automatisée **80** est ici insérée dans le canal de purge **84.** Alors, la liaison entre la seringue automatisée et le canal de purge est étanche. Le risque de fuite lors d'un passage d'urine depuis la seringue automatisée vers le canal de purge est réduit.

De préférence, le canal de purge **84** est hydrophobe, permettant une meilleure évacuation de l'urine. Alors, le risque de contamination de l'urine acheminé entre deux collectes d'urine successives est réduit.

La pompe **86** est agencée entre une première portion **82a** et une deuxième portion **82b** du canal de collecte **82.**

La pompe **86** peut aspirer de l'urine depuis l'orifice de collecte **32.** La pompe aspire par exemple entre 5 microlitres et 1 mL, de préférence environ 20 microlitres. De plus, la pompe permet d'acheminer un volume suffisant d'urine vers l'injecteur **46** pour pouvoir réaliser une analyse concluante. Un débit d'aspiration de la pompe **86** est choisi en fonction du diamètre de l'orifice de collecte. Avantageusement, la pompe peut aspirer de l'urine depuis l'orifice de collecte vers l'injecteur sans former de bulles d'air.

Selon une autre phase, après miction et hors séquence de chasse d'eau, la pompe **86** peut également aspirer de l'air depuis l'orifice de collecte **32.** L'air traverse alors le moyen d'acheminement d'urine **48** et la seringue automatisée **80** jusqu'à l'orifice de vidange **34.** Alors, la pompe permet d'expulser de l'urine ou de l'eau contenue dans le dispositif d'analyse d'urine. De l'urine collectée pour une analyse est alors protégée d'une éventuelle contamination par l'eau des toilettes ou d'une collecte précédente.

Selon une autre phase ou une variante de réalisation, il peut être prévu que la pompe **86** puisse aspirer de l'eau lors de l'activation de la chasse d'eau des toilettes pour évacuer l'urine contenue dans dispositif d'analyse d'urine.

La pompe **86** peut être de différents types possibles. La pompe **86** peut être une pompe péristaltique miniaturisée. La pompe **86** peut être un système de pompe pneumatique miniaturisée comme détaillé ci-après.

Dans le cas où la pompe **86** est une pompe pneumatique miniaturisée, ce système pneumatique est configuré pour créer une dépression afin d'aspirer l'urine depuis l'orifice de collecte **32,** puis une pression positive pour pousser l'urine vers l'injecteur **46** et le canal de purge. En outre, ce système pneumatique comprend un espace tampon interne interposé entre la première portion **82a** et la deuxième portion **82b** du canal de collecte **82,** un clapet d'amission (clapet anti-retour) et un clapet de refoulement (clapet anti-retour).

Lorsque ce système pneumatique créée une dépression, cela aspire du fluide depuis l'orifice de collecte **32** via la première portion **82a** et le clapet d'admission; le fluide aspiré peut être alors, selon les cas, de l'urine et/ou de l'eau et/ou de l'air.

Le fluide ainsi aspiré est stocké dans l'espace tampon interne du système pneumatique. Ensuite, ce système pneumatique créée une surpression, et cela pousse du fluide, à partir de l'espace tampon interne, au travers du clapet de refoulement et de la deuxième portion **82b,** en direction de l'injecteur **46.**

Grâce aux clapets anti-retour, le système pneumatique peut être dépourvu de valves contrôlées, ce qui réduit la complexité le dispositif d'analyse d'urine.

La pompe du système pneumatique peut être ici une pompe de type rotative, le sens de rotation fournissant respectivement et sélectivement une dépression ou une surpression. La pompe du système pneumatique peut être aussi une pompe de type piézoélectrique.

Lorsque de l'urine est aspirée, il peut être prévu que l'injecteur soit en position de purge pour évacuer de l'air jusqu'à ce que de l'urine parvienne au seuil de l'aiguille.

La solution présentée permet de contrôler de manière précise le volume acheminé dans le dispositif d'analyse d'urine.

### Variante pompe et canalisation

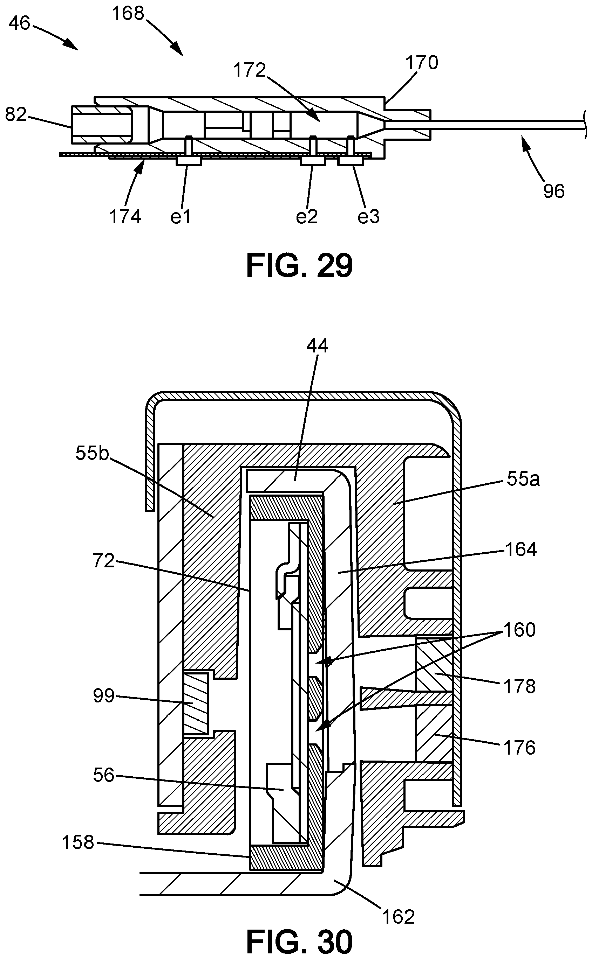

On décrit par la suite un deuxième exemple de moyen d'acheminement d'urine 48 en référence à la figure 29.

Dans cet exemple, l'injecteur 46 est radialement interne par rapport au support rotatif 44. La direction de translation de la seringue de l'injecteur peut présenter un angle (par exemple, entre 5° et 45° ou entre 5° et 30°) par rapport à une direction radiale qui passe par l'extrémité de la seringue en position rétractée) pour des raisons d'encombrement L'injecteur 46 peut accéder directement à l'orifice de vidange 34 au travers l'ouverture 74 du support rotatif 44. Le moyen d'acheminement d'urine peut être dépourvu du canal de purge 84. L'évacuation d'urine peut se faire par injection au travers l'orifice de vidange 34.

En outre, dans cet exemple, la pompe 86 est également radialement interne par rapport au support rotatif. L'agencement de la pompe 86 et de l'injecteur 46 permet de libérer l'espace extérieur au support rotatif 44, de sorte que le diamètre D44 du support rotatif 44 peut être augmenté. Un nombre augmenté de bandelettes de tests 56 peut être stocké par le support rotatif 44.

### Moyen de contrôle d'urine

Comme visible à la figure 29, l'injecteur 46 _{.}peut comprendre un moyen de contrôle d'urine 168. Le moyen de contrôle d'urine 168 est destiné à permettre une mesure de volume et de débit d'urine circulant dans l'aiguille 96.

Le moyen de contrôle d'urine 168 comprend une douille 170. La douille 170 est agencée entre l'aiguille 96 et le canal de collecte d'urine 82. La douille 170 est une pièce cylindrique creuse. La douille 170 définit une cavité interne 172 au travers de laquelle l'urine peut circuler pour rejoindre l'aiguille 96.

Un circuit imprimé « *flexible printed circuit »* 174 est monté sur une paroi extérieure de la douille 170. Trois électrodes conductrices e1, e2, e3 s'étendent depuis le circuit imprimé 174 pour déboucher dans la cavité 172. Les électrodes peuvent détecter de l'urine circulant dans la cavité 172 par génération d'un signal électrique.

Un volume de référence est défini par la distance entre une première électrode e1, au voisinage de l'aiguille 96, et une deuxième électrode e2, distante de l'aiguille 96, et la section de la cavité 172. Le volume de référence est par exemple 20µL. La pompe 86 peut être activée pour acheminer de l'urine vers l'aiguille 96 jusqu'à ce que de l'urine soit en contact à la fois avec les première et deuxième électrodes e1, e2. Lorsque de l'urine est à la fois en contact avec les premières et deuxièmes électrodes e1, e2, un signal électrique peut circuler en circuit fermé entre les premières et deuxièmes électrodes e1, e2. Le circuit fermé indique que le volume de référence est atteint dans la cavité.

On note que le volume de référence est supérieur au volume contrôlé d'urine destiné à être injecté sur une bandelette de test 56.

Le volume de référence comprend un volume de pré-injection. Le volume de pré-injection peut être circulé au travers de l'aiguille 96 pour rejoindre l'orifice de vidange 34. Le volume de pré-injection permet de s'assurer que l'aiguille 96 est chargée en urine avant une analyse. On s'assure que l'urine dans l'aiguille 96 ne comporte pas de bulles d'air qui pourrait réduire le volume d'urine réellement injecté sur la bandelette de test 56.

Le volume de référence comprend également un volume de sécurité permettant de prendre en compte les tolérances électromagnétiques des électrodes e1, e2, e3.

En outre, lorsque la pompe 86 est activée, le temps s'écoulant entre un contact de l'urine avec une troisième électrode e3, disposée entre une extrémité de la douille 170 reliée à l'aiguille 96 et la première électrode e1, permet de mesurer le débit d'urine. Le débit d'urine peut déterminer le temps d'activation de la pompe 86 nécessaire à l'injection du volume contrôlé d'urine sur la bandelette de test 56. En effet, la relation entre le débit d'urine et le temps d'activation de la pompe 86 est linéaire. La détermination du temps d'activation permet de s'affranchir du besoin de calibrer la pompe 86.

### Autres fonctions supportées par le dispositif

Dans un mode de réalisation, l'ensemble de test **24** comporte un capteur de présence d'urine **38.** Le capteur de présence d'urine est agencé à proximité de l'orifice de collecte. Le capteur de présence d'urine permet alors de détecter lorsque de l'urine est présente au voisinage de l'orifice de collecte.

Selon un mode de réalisation, le capteur de présence d'urine 38 pourrait former un anneau autour de l'orifice de collecte. L'intégration du capteur de présence d'urine dans le dispositif d'analyse d'urine est alors discrète.

Le capteur de présence d'urine **38** peut un capteur de température, par exemple une thermistance. Le capteur de température permet en effet de distinguer entre de l'urine et de l'eau provenant des toilettes. En outre, le capteur de température peut également être exploité pour mesurer la température de l'urine. La température de l'urine permet notamment de détecter des périodes de fécondité, par comparaison à une ou plusieurs courbes de référence. L'utilisation d'un capteur de température permet alors de réduire le nombre de composants exploités par l'ensemble de test pour réaliser une analyse. La complexité et les coûts liés à la fabrication du dispositif d'analyse d'urine sont réduits.

Alternativement, le capteur de présence d'urine **38** peut être tout type de détecteur de liquide, par exemple un capteur de type capacitif ou résistif. Alors, un capteur de température est distinct du capteur de présence d'urine. Le capteur de température peut être dédié à la mesure de la température de l'urine, notamment pour détecter une période de fécondité.

### Système d'analyse

Le système d'analyse **50** réalise l'analyse par colorimétrie sur les bandelettes de test. On entend par « analyse par colorimétrie » une mesure d'absorbance ou de fluorescence sous un éclairage prédéterminé, en transmission on en réflexion. Le système d'analyse peut alors déterminer un ou plusieurs résultats d'analyses.

Le système d'analyse **50** est ici agencé radialement à l'extérieur du support rotatif. Alternativement, par exemple lorsque les bandelettes de test sont stockées sur une face interne du support rotatif, le système d'analyse pourrait être agencé radialement à l'intérieur du support rotatif. Dans un exemple particulier décrit plus loin, le système d'analyse 50 chevauche le support rotatif 44.

En variante, le système d'analyse **50** peut être agencé sur un moteur linéaire. Moyennant quoi, le système d'analyse peut se rapprocher d'une bandelette de test pour effectuer une analyse plus précise. Cette configuration apparait particulièrement intéressante lorsque le dispositif d'analyse d'urine comporte deux supports rotatifs. En effet, le système d'analyse peut effectuer une analyse concluante sur une bandelette de test stockée dans le support rotatif radialement interne **442.**

Le système d'analyse **50** peut comporter une source de lumière, par exemple une ou plusieurs diodes électroluminescentes (LED). La ou les sources de lumière illuminent une bandelette de test. Pour simplifier le reste de la description, la source de lumière sera une LED.

De manière préférée, le système d'analyse **50** comporte plusieurs LEDs de longueur d'onde spécifique aux différents réactifs contenus dans différentes types de bandelettes de test. Ainsi, le dispositif d'analyse d'urine peut réaliser différentes analyses avec précision.

En variante, le système d'analyse **50** peut comporter une unique LED. Par exemple, la LED peut être blanche. Alors, la LED peut couvrir tout le spectre du visible. Cette configuration permet de réduire la complexité du système d'analyse.

Le système d'analyse **50** peut également comporter un collimateur. Le collimateur permet d'orienter l'illumination de la ou des LEDs vers la bandelette de test.

Le système d'analyse **50** comporte également le capteur optique 99. Le capteur optique **99** est notamment de type photodiode, CCD (« *Charged Coupled Device* ») ou CMOS (« *Complementary Metal Oxide Semiconductor* »). Alors, le capteur optique peut mesurer l'absorbance ou la fluorescence du réactif de la bandelette de test, notamment par transmission ou réflexion, pour établir le ou les résultats d'analyses.

Le capteur optique **99** peut être surmonté d'un filtre. Le filtre permet d'augmenter la sensibilité du capteur optique à des longueurs d'ondes spécifiques. La précision d'une analyse est alors très satisfaisante.

On décrit par la suite un mode de réalisation particulier du système d'analyse 50, illustré à la figure 30. Le système d'analyse 50 décrit ci-après est particulièrement adapté au support rotatif 44 stockant les bandelettes de test 56 dans une paroi interne 44b du support rotatif 44.

Le système d'analyse 50 est ici agencé de part et d'autre du support rotatif 44 lorsqu'il est inséré dans le boitier 22. Le système d'analyse 50 chevauche le support rotatif 44 inséré dans le boitier 22. Une première partie 50a du système d'analyse 50 est radialement externe au support rotatif 44 et une deuxième partie 55b du système d'analyse 50 est radialement interne au support rotatif 44. Le système d'analyse 50 opère par transmission de la lumière depuis la première partie 55a vers la deuxième partie 55b.

La première partie 55a comprend au moins une source de lumière, par exemple sous la forme d'une paire de LEDs 176, 178. Chaque LED de la paire est alignée avec un trou 160 prévu sur le séparateur 158 du support rotatif 44. La lumière est guidée au travers des trous 160 pour éclairer les bandelettes de test 56. Une première LED 176 de la paire est de couleur blanche pour couvrir l'ensemble du spectre visible et déterminer des changements de couleur de la bandelette de test 56. Une deuxième LED 178 de la paire est monochromatique, par exemple ultraviolette, pour exciter des fluorophores et permettre l'observation de leur longueur d'onde d'émission.

Le système d'analyse 50 pourrait comprendre une paire de LEDs ou deux paires de LEDs voisines. Selon le nombre de paires de LEDs, il apparaît possible d'analyser plusieurs bandelettes de test 56 simultanément.

La deuxième partie 55b comprend le capteur optique 99. Le capteur optique 99 est ici de type spectral. Il comporte plusieurs photodiodes surmontées de filtres permettant de mesurer l'intensité de la lumière à différentes longueurs d'ondes réparties dans le visible. Le capteur 99 est compatible avec les mesures optiques par absorbance et par fluorescence.

Le capteur 99 peut être utilisé pour différents types de bandelettes de test. Dans l'exemple de la figure 30, la bandelette de test 56 est du type immuno-chromatographique et comporte une zone de test et une zone de contrôle alignées respectivement avec les trous 160, dont le changement de couleur permet d'obtenir un résultat. Dans un autre exemple, la bandelette de test 56 peut être une bandelette colorimétrique et comporter deux zones de test distinctes (pour tester par exemple simultanément le pH et la gravité spécifique de l'urine) alignées respectivement avec les trous 160.

On notera que d'autres configurations avec par exemple plus de deux trous 160 sont envisageables pour augmenter par exemple le nombre de tests réalisées avec une même bandelette.

### Communication et aspects système

Le dispositif d'analyse d'urine comporte un module de communication **41.** La communication est sans-fil. Le module de communication **41** exploite un réseau local, par exemple de type Bluetooth, Bluetooth Low-Energy (BLE) ou Wi-fi. Le réseau local permet de préserver une batterie **94** de l'ensemble de test **24.** Ainsi, l'autonomie du dispositif d'analyse est augmentée.

En variante, le module de communication **41** peut exploiter un réseau de télécommunication cellulaire. Le réseau de communication cellulaire peut par exemple être GSM, 3G, 4G, 5G,4G-LTE. Le module de communication **41** a alors une portée plus longue.

Alternativement encore, le module de communication **41** peut exploiter une passerelle connectée au réseau de télécommunications cellulaire. La passerelle peut notamment être un routeur, par exemple un routeur Wi-fi connecté au réseau cellulaire, un hub, c'est-à-dire un dispositif connecté directement au réseau cellulaire, ou le smartphone de l'utilisateur. Alors, le dispositif d'analyse d'urine peut être connecté au réseau cellulaire sans compromettre l'autonomie de la batterie **94.**

De préférence, le module de communication 41 exploite la technologie Bluetooth Low Energy (BLE) pour communiquer avec un smartphone 61 de l'utilisateur et la technologie Wifi pour se connecter à un serveur distant 98.

Le module de communication **41** permet de déclencher une analyse, via une commande à distance.

De manière préférée, l'utilisateur peut lancer une analyse directement depuis le smartphone **61.** L'utilisateur a le contrôle sur le dispositif d'analyse d'urine, et peut choisir lorsqu'il souhaite réaliser une analyse, ainsi que le type d'analyse qu'il souhaite réaliser. En outre, l'utilisateur peut être identifié par le smartphone. L'analyse effectuée peut être adaptée à l'utilisateur identifié, et les résultats envoyés pour enrichir l'historique de cet utilisateur identifié.

En variante, l'analyse est lancée par communication avec un appareil déporté **42** à proximité des toilettes.

L'appareil déporté comporte un bouton **55.** L'utilisateur peut alors appuyer sur le bouton pour lancer une analyse. L'utilisateur a le contrôle sur le dispositif d'analyse d'urine, et peut choisir lorsqu'il souhaite réaliser une analyse.

Le bouton **55** peut être pourvu d'un capteur biométrique **57.** Le bouton peut alors identifier l'utilisateur appuyant sur le bouton. Alors, une analyse pertinente à l'utilisateur identifié peut être effectuée par le dispositif d'analyse d'urine. En outre, l'analyse effectuée peut être choisie en fonction de l'utilisateur identifié, et les résultats envoyés pour enrichir l'historique de cet utilisateur identifié.

L'appareil déporté **42** comporte également un afficheur **59.** Par exemple, l'afficheur peut consister en une ou plusieurs diodes électroluminescentes (LED) de couleurs. L'afficheur peut également comprendre un écran. L'afficheur permet d'informer l'utilisateur. Par exemple, l'utilisateur peut être informé qu'un appui sur le bouton a été perçu, et/ou que l'utilisateur a été identifié et/ou qu'une analyse va être réalisée.

Alternativement, l'appareil déporté **42** peut être un bracelet connecté associé à l'utilisateur. Dans ce cas, l'utilisateur peut être automatiquement détecté lorsqu'il est à proximité des toilettes. L'utilisateur peut également être identifié par le bracelet connecté. Ainsi, une analyse peut être lancée automatiquement, sans action de l'utilisateur. À noter que le bracelet connecté peut se présenter sous la forme d'une montre connectée.

Le module de communication **41** permet également de communiquer le ou les résultats d'analyses. Le ou les résultats d'analyses peuvent être une ou plusieurs grandeur(s) révélant l'un(e) parmi : une période de fécondité, une grossesse, des infections urinaires, des problèmes de foie, des insuffisances rénales, une lithiase urique, une déshydratation, une maladie cardiaque et/ou un diabète. Le ou les résultats peuvent également être un indicateur de l'observance médicamenteuse.

Le module de communication **41** peut envoyer le ou les résultats d'analyses directement à l'afficheur **59** ou au smartphone **61.** Le module de communication peut alors être dépourvu d'une connexion au réseau de télécommunication cellulaire. Le ou les résultats d'analyses peuvent être interprétés localement par l'unité électronique de commande **45,** ou par l'application du smartphone. Ainsi, les coûts d'exploitation du dispositif d'analyse d'urine sont réduits.

Alternativement, le module de communication **41** peut envoyer le ou les résultats vers un serveur **98** distant. Le serveur distant peut interpréter le ou les résultats d'analyses. L'exploitation d'un serveur permet de réduire les capacités de calcul nécessaire localement pour l'interprétation du ou des résultats d'analyses.

Le serveur distant **98** peut également être pourvu d'une capacité de stockage. Alors, le serveur distant peut stocker le ou les résultats d'une pluralité d'analyses successives, pour un (ou chaque) utilisateur.

L'utilisateur peut visualiser et exploiter un ou plusieurs résultats d'analyses, reçu(s) directement du dispositif d'analyse ou du serveur disant. Par exemple, l'utilisateur peut visualiser et exploiter les résultats depuis l'application du smartphone. Alternativement, l'utilisateur peut accéder à un site web depuis un ordinateur, pour accéder ces données de résultat.

Par ailleurs, le boitier 22 peut directement présenter des informations à l'utilisateur. Le boitier 22 peut présenter un témoin lumineux 194, notamment une ou plusieurs diodes électroluminescentes (LED). Le témoin lumineux 194 peut permettre une communication entre le boitier 22 et l'utilisateur. Différentes couleurs d'affichage peuvent faire passer des messages précis. L'allumage du témoin en rouge peut, par exemple, signifier un niveau de batterie 98 faible. Le témoin lumineux 194 peut également indiquer à l'utilisateur que le boitier 22 est correctement positionné sur l'élément de fixation 61.

Enfin, un bouton 192 peut être accessible à l'utilisateur depuis le boitier 22. Le bouton 192 est accessible à l'utilisateur pour réinitialiser le dispositif d'analyse d'urine 12. Le bouton 192 peut notamment être accessible à l'utilisateur lorsque le capot amovible 90 est retiré.

### Unité de commande

L'unité de commande électronique 45 peut être divisée en une pluralité de sous circuits (voir par exemple la figure 31). Les sous circuits permettent une flexibilité quant à leur arrangement dans le boitier 22 du dispositif d'analyse.

Un circuit principal 180 peut comprendre un microcontrôleur principal. Le circuit principal 180 peut commander les moteurs 76, 91, la pompe 86 et le module de communication 41. Le circuit principal 180 est également apte à coopérer avec les autres circuits pour permettre la coordination des autres circuits.

Un circuit d'analyse optique 182 peut coopérer avec le capteur optique 99 du système d'analyse 50. Le circuit d'analyse optique 182 peut être connecté à un premier circuit de diodes 184. Le premier circuit de diode 184 peut commander la ou les LEDs du système d'analyse 50 pour analyser par colorimétrie les bandelettes de tests 56.

Un circuit de témoin lumineux 186 peut commander le témoin lumineux 194 pour la communication avec l'utilisateur depuis le boitier 22.

Un circuit de détection de volume et de débit 188 peut comprendre le circuit imprimé 174 comportant les électrodes e1, e2, 23. Le circuit de détection de volume et de débit 188 est sensible aux signaux issus des électrodes lorsqu'elles sont au contact d'urine.

Un circuit annexe 196 peut permettre l'accès à une interface de test et de diagnostic, permettant des tests de qualité du dispositif d'analyse d'urine 12. Le circuit annexe 196 peut être relié à un circuit de réinitialisation 190 comprenant le bouton 192 de réinitialisation du dispositif d'analyse d'urine 12.

Plus particulièrement, le circuit principal comprend un premier système sur puce (SOC ou "System on Chip") Bluetooth Low Energy. Le premier système sur puce permet la communication avec le smartphone 61 et gère les autres circuits. Le premier système sur puce coopère avec un deuxième système-sur-puce Wifi (812.11) qui prend en charge l'échange de données avec le serveur 98. Cette architecture permet d'optimiser la consommation de la batterie 94 en maintenant éteints le deuxième système sur puce, fortement consommateur en énergie, lorsqu'il n'est pas utilisé.

L'unité de commande électronique 45 est alimentée par la batterie 94 prévue à l'intérieur du boitier 22. La batterie 94 est de type lithium-ion. La capacité de la batterie est d'environ 1080mAh. Une telle capacité permet d'assurer une autonomie satisfaisante du dispositif sans compromettre les dimensions du boitier.

La batterie 94 comprend des connecteurs de charge. Les connecteurs de charge sont accessibles depuis l'extérieur du boitier 22 pour permettre une recharge de la batterie 94. Par exemple, le boitier 22 peut être placé sur un socle pour relier les connecteurs de charge à une source d'énergie. Alternativement, une recharge par induction pourrait également être envisagée.

### Aspects de procédé

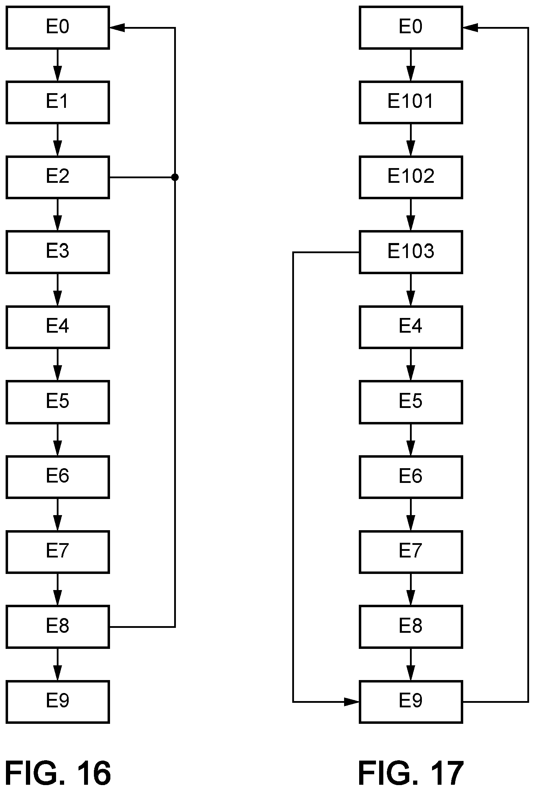

Par la suite, on décrit plus en détail deux procédés permettant de réaliser une analyse exploitant le dispositif d'analyse d'urine décrit ci-avant, en référence à la figure 16 et la figure 17. Les procédés sont réalisés par l'unité de commande électronique **45.** Tel qu'illustré à la figure 15, l'unité de commande électronique 45 reçoit des informations des capteurs **38, 99** et pilote les moteurs **76, 91** ainsi que la pompe **86.**

Le premier procédé, illustré à la figure 16, consiste à lancer une analyse suite à une demande de l'utilisateur.

Au préalable, on note que le boitier 22 est positionné dans les toilettes. Le support rotatif 44 comporte au moins une bandelette de test 56 non-utilisée et la batterie 98 du dispositif est suffisamment chargée. Si tel n'est pas le cas, le dispositif indique à l'utilisateur que l'analyse ne peut pas être réalisée.

L'étape **E0** consiste à maintenir le dispositif d'analyse d'urine dans une position de purge. L'ouverture **74** du support rotatif est alignée avec la seringue automatisée **80** de l'injecteur **46.** La seringue automatisée traverse l'ouverture du support rotatif. La seringue automatisée est insérée dans le canal de purge ou accède directement à l'orifice de vidange 34. Ainsi, de l'urine ou de l'eau reçu des toilettes dans le canal de collecte **82** peut rejoindre l'orifice de vidange **34.**

A l'étape **E1,** le dispositif d'analyse d'urine reçoit la demande d'analyse d'urine. La demande d'analyse peut provenir de l'appui sur le bouton **55** par l'utilisateur. La demande d'analyse peut également être commandée depuis le smartphone **61.** La demande d'analyse peut également être réalisée automatiquement, lorsque l'utilisateur est à proximité des toilettes. L'afficheur **59** peut indiquer à l'utilisateur que son appui a été reçu. L'afficheur peut également indiquer si un utilisateur a été reconnu lors de l'appui sur le bouton.

A l'étape **E2,** le dispositif d'analyse d'urine attend de détecter de l'urine à proximité de l'orifice de collecte. Le boitier peut en outre être équipé de LEDs pour alerter un utilisateur que le dispositif d'analyse d'urine attend de détecter de l'urine. Les LEDs peuvent être celles du témoin lumineux 194.

Si aucun jet d'urine n'est reçu dans un délai imparti, alors le dispositif d'analyse d'urine retourne à l'étape **E0.**

Si, au contraire, un jet d'urine est détecté, l'étape **E3** consiste à activer la pompe **86** pour acheminer l'urine depuis l'orifice de collecte vers la seringue automatisée **80.** L'étape **E3** s'arrête lorsque suffisamment d'urine est détectée dans l'espace buffer interne ou dans la seringue automatisée pour réaliser une analyse. Si l'injecteur 46 comprend le moyen de contrôle d'urine 168, l'étape E3 s'arrête lorsque le volume de référence délimité par les électrodes e1 et e2 est détecté dans la cavité 172 de la douille 170.

En outre, lorsque l'injecteur 46 comprend le moyen de contrôle d'urine 168, l'étape E3 comprend calculer le débit d'urine circulant entre les électrodes e1 et e3. Le débit d'urine permet de déterminer le temps d'activation de la pompe nécessaire à l'injection d'un volume précis.

En outre, lorsque l'injecteur 46 comprend le moyen de contrôle d'urine, l'étape E3 peut comprendre réactiver la pompe pour expulser le volume de pré-injection vers l'orifice de vidange 34. L'aiguille 96 est alors dépourvue d'air, qui, de par sa compressibilité, peut impacter le temps d'activation de la pompe nécessaire à l'injection du volume précis.

L'étape **E4** consiste à positionner le dispositif d'analyse d'urine dans une position de pré-injection. La position de pré-injection prépare le dispositif d'analyse d'urine à une injection d'urine sur une bandelette de test. La seringue automatisée **80** est rétractée du canal de purge ou de l'orifice de vidange 34 et de l'ouverture **74** du support rotatif, ou bien elle était déjà dans une position rétractée. Le support rotatif est tourné pour qu'un logement du support rotatif soit face à la seringue automatisée.

Il faut noter que les étapes **E3** et **E4** peuvent être réalisées simultanément.

L'étape **E5** consiste par la suite à positionner le dispositif d'analyse d'urine dans une position d'injection. La seringue automatisée **80** est translatée grâce à l'action du moteur **91,** pour percer l'opercule **72.** La seringue automatisée injecte de l'urine sur une bandelette de test. L'urine injectée peut alors réagir avec les réactifs de la bandelette de test. Après l'injection effective, l'aiguille peut être rétractée.

L'injection d'urine à l'étape E5 peut se faire en deux temps. Par exemple, la seringue automatisée 80 peut injecter deux fois entre 2,5 microlitres et 3,5 microlitres. Cette solution permet de prendre en compte un temps de réaction et de migration de l'urine sur la bandelette de test 56.

Lorsque le dispositif d'analyse d'urine comprend le moyen de contrôle d'urine 168, l'injection d'urine peut être effectuée en activant la pompe pour la durée déterminée à l'étape E3. On s'assure ainsi qu'un volume précis et répétable est injecté sur la bandelette de test 56.

L'étape **E6** consiste à positionner le dispositif d'analyse d'urine dans la position d'analyse. On vérifie d'abord que la seringue automatisée est rétractée du logement. La seringue automatisée retrouve la position de pré-injection. Le support rotatif tourne pour positionner la bandelette de test ayant reçu l'urine à l'étape **E4** face au système d'analyse.

L'étape **E7** consiste à établir le ou les résultats d'analyse. Le système d'analyse **50** réalise une analyse par colorimétrie sur la bandelette de test. Le dispositif d'analyse d'urine en déduit le ou les résultats d'analyses. L'analyse réalisée dépend du type de bandelette de test. L'analyse réalisée peut également dépendre d'un choix de l'utilisateur. L'analyse effectuée peut également être choisie en fonction de l'utilisateur identifié.

L'étape **E8** correspond à la transmission du ou des résultats. Le ou les résultats peuvent être transmis directement à l'utilisateur. Le ou les résultats peuvent également être envoyés vers le serveur **98.** L'utilisateur peut par exemple visualiser et exploiter le ou les résultats sur l'application du smartphone **61,** ou sur un site web. Le ou les résultats peuvent également être envoyés vers un professionnel de santé.

L'étape **E9** consiste à purger le dispositif d'analyse d'urine. La pompe **86** est activée pour pousser de l'air dans le moyen d'acheminement **48.** Alors, l'urine est expulsée du dispositif d'analyse d'urine via le canal de purge et par l'orifice de vidange **34.** Le dispositif d'analyse d'urine retourne ensuite dans la position de purge de l'étape **E0.**

Il est à noter que les étapes **E4** à **E7** peuvent être répétées plusieurs fois. Ainsi, plusieurs bandelettes de test reçoivent de l'urine et sont analysées. Alors, plusieurs analyses peuvent être réalisées à partir d'une unique aspiration d'urine à l'étape **E3.**

En variante, dans l'exemple illustré à la figure 17, le procédé est initié lorsque de l'urine est détectée sur le boitier.

L'étape **E0** consiste à maintenir le dispositif d'analyse d'urine dans la position de purge, telle que décrite ci-avant.

L'étape **E101** consiste à détecter la présence d'urine à proximité de l'orifice de collecte 32.

Alors, l'étape **E102** consiste à activer la pompe **86** pour aspirer l'urine depuis l'orifice de collecte **32** vers la seringue automatisée **80.** L'étape **E3** s'arrête lorsque suffisamment d'urine est détectée dans la seringue automatisée et/ou dans le buffer tampon interne pour réaliser une analyse. L'étape E3 peut également s'arrêter lorsque suffisamment d'urine est détectée par le moyen de contrôle d'urine 168. L'étape E3 peut également comprendre la détermination du débit d'urine et la réactivation de la pompe comme décrit plus haut. L'afficheur **59** peut indiquer qu'une analyse est prête à être réalisée. En variante, le boitier, pourvu de LEDs, peut indiquer qu'une analyse est prête à être réalisée.

A l'étape **E103,** le dispositif d'analyse d'urine attend la réception d'une demande d'analyse d'un utilisateur. La demande d'analyse peut provenir de l'appui sur le bouton **55** par l'utilisateur. La demande d'analyse peut également être commandée depuis le smartphone **61.**

Si la demande d'analyse n'est pas reçue dans un délai imparti, alors la pompe est activée pour purger le dispositif d'analyse d'urine de l'urine collectée (Etape **E9**). Le dispositif d'analyse d'urine retourne à la position de purge de l'étape **E0.**

Si au contraire la demande d'analyse est reçue, alors une analyse est réalisée. Le dispositif d'analyse d'urine réalise alors les étapes **E4** à **E9** tels que décrites précédemment.

## Revendications

1. Dispositif d'analyse d'urine (12) destiné à être positionné à l'intérieur de toilettes comprenant un ensemble de test (24), lequel comporte :
- au moins un support rotatif (44) comprenant une pluralité de bandelettes de test (56) ;
- un injecteur (46) configuré pour injecter un volume contrôlé d'urine sur au moins une des bandelettes de test ;
- un système d'analyse (50) configuré pour détecter un résultat d'injection d'urine sur la bandelette de test ;
**caractérisé en ce que** les bandelettes de test (56) de la pluralité de bandelettes de test (56) sont fixées sur le support rotatif et **en ce que** l'injecteur (46) comporte une seringue (80) mobile configurée pour être rapprochée ou éloignée d'une bandelette de test (56) .

2. Dispositif d'analyse d'urine (12) selon la revendication précédente, dans lequel le ou chaque support rotatif (44) est configuré pour entrainer en rotation une bandelette de test (56) et la présenter sélectivement devant l'injecteur (46) et/ou devant le système d'analyse (50).

3. Dispositif d'analyse d'urine (12) selon l'une quelconque des revendications précédentes, dans lequel chaque support rotatif (44) comporte des logements (54) recevant une ou plusieurs bandelettes de test (56).

4. Dispositif d'analyse d'urine (12) selon la revendication précédente, dans lequel les logements (54) sont fermés par au moins un opercule (72), par exemple transparent ou translucide.

5. Dispositif d'analyse d'urine (12) selon la revendication 3 ou 4, dans lequel le support rotatif (44) est cylindrique, et les logements (54) sont agencés sur une paroi interne (44b) du support rotatif (44).

6. Dispositif d'analyse d'urine (12) selon l'une des revendications 3 à 5, dans lequel le support rotatif comprend un séparateur (158) comportant les logements (54), le séparateur (158) étant en matériau flexible, de préférence en élastomère, et un réceptacle (156) recevant le séparateur (158).

7. Dispositif d'analyse d'urine (12) selon la revendication précédente, dans lequel le réceptacle (156) comprend une portion annulaire (162) et une portion cylindrique (164), radialement externe à la portion annulaire (162).

8. Dispositif d'analyse d'urine (12) selon la revendication précédente, dans lequel le séparateur (158) comprend des trous (160) débouchant dans les logements (54), et la portion cylindrique (164) au contact du séparateur (158) est transparente, de sorte que le système d'analyse (50) détecte un résultat d'analyse par transmission de lumière au travers la portion cylindrique (164) et les trous (160) du séparateur (158).

9. Dispositif d'analyse d'urine (12) selon l'une quelconque des revendications 1 à 8, dans lequel les bandelettes sont réparties le long d'un cercle ou d'un arc de cercle autour d'un axe (A) de rotation du support rotatif (44), et, optionnellement, les bandelettes s'étendent chacune parallèlement à l'axe (A).

10. Dispositif d'analyse d'urine (12) selon l'une quelconque des revendications 1 à 9, dans lequel le système d'analyse comprend :
- au moins une source de lumière (LED ; 176, 178) disposée d'une part du support rotatif (44), l'au moins une source de lumière émettant de la lumière en direction du support rotatif (44), et,
- un capteur (99) disposé d'autre part du support rotatif (44), le capteur (99) faisant face à l'au moins une source de lumière de sorte à détecter la lumière traversant le support rotatif (44).

11. Dispositif d'analyse d'urine (12) selon l'une quelconque des revendications 1 à 10, dans lequel un boitier (22) renferme l'ensemble de test (24), le boitier étant configuré pour être entièrement positionné à l'intérieur de toilettes (10).

12. Dispositif d'analyse d'urine (12) selon l'un quelconque des revendications 1 à 11, dans lequel le support rotatif est agencé de manière amovible dans le boitier (22).

13. Dispositif d'analyse d'urine (12) selon l'une quelconque des revendications 1 à 12, dans lequel l'ensemble de test (24) comprend en outre un capteur de présence d'urine (38), le capteur étant par exemple un capteur de température.

14. Dispositif d'analyse d'urine (12) selon l'une quelconque des revendications 1 à 13, dans lequel l'injecteur (46) est agencé dans une zone radialement interne au support rotatif (44).

15. Dispositif d'analyse d'urine (12) selon l'une quelconque des revendications 1 à 14, en combinaison avec la revendication 10 dans lequel le capteur (99) est configuré pour contrôler la position angulaire du support rotatif par la détection d'un ou plusieurs marqueurs sur le support rotatif.

## Patentansprüche

1. Urintestvorrichtung (12) zur Positionierung in einer Toilette, die eine Testanordnung (24) umfasst, die Folgendes umfasst:
- mindestens einen drehbaren Träger (44), der eine Vielzahl von Teststreifen (56) umfasst;
- einen Injektor (46), der so konfiguriert ist, dass er ein kontrolliertes Volumen an Urin auf mindestens einen der Teststreifen injiziert;
- ein Analysesystem (50), das so konfiguriert ist, dass es ein Ergebnis der Injektion von Urin auf den Teststreifen erfasst;
**dadurch gekennzeichnet, dass** die Teststreifen (56) der Vielzahl von Teststreifen (56) an der drehbaren Halterung befestigt sind und dass die Injektionsvorrichtung (46) eine bewegliche Spritze (80) umfasst, die so konfiguriert ist, dass sie zu einem Teststreifen (56) hin oder von ihm weg bewegt werden kann.

2. Urinanalysevorrichtung (12) nach dem vorhergehenden Anspruch, wobei der oder jeder drehbare Träger (44) so konfiguriert ist, dass er einen Teststreifen (56) in Drehung versetzt und ihn selektiv vor dem Injektor (46) und/oder vor dem Analysesystem (50) präsentiert.

3. Urinanalysevorrichtung (12) nach einem der vorhergehenden Ansprüche, wobei jede drehbare Halterung (44) Aufnahmen (54) aufweist, die einen oder mehrere Teststreifen (56) aufnehmen.

4. Urinanalysevorrichtung (12) nach dem vorhergehenden Anspruch, wobei die Aufnahmen (54) durch mindestens einen Deckel (72) verschlossen sind, der beispielsweise durchsichtig oder durchscheinend ist.

5. Urinanalysevorrichtung (12) nach Anspruch 3 oder 4, wobei der drehbare Träger (44) zylindrisch ist und die Aufnahmen (54) an einer Innenwand (44b) des drehbaren Trägers (44) angeordnet sind.

6. Urinanalysevorrichtung (12) nach einem der Ansprüche 3 bis 5, wobei der Drehhalter einen Separator (158) mit den Aufnahmen (54), wobei der Separator (158) aus einem flexiblen Material, vorzugsweise aus Elastomer, besteht, und einen Behälter (156), der den Separator (158) aufnimmt, umfasst.

7. Urinanalysevorrichtung (12) nach dem vorhergehenden Anspruch, wobei das Behältnis (156) einen ringförmigen Abschnitt (162) und einen zylindrischen Abschnitt (164), der radial außerhalb des ringförmigen Abschnitts (162) liegt, umfasst.

8. Urinanalysevorrichtung (12) nach dem vorhergehenden Anspruch, wobei der Separator (158) Löcher (160) aufweist, die in die Gehäuse (54) münden, und der zylindrische Abschnitt (164), der den Separator (158) berührt, transparent ist, so dass das Analysesystem (50) ein Analyseergebnis durch Lichtübertragung durch den zylindrischen Abschnitt (164) und die Löcher (160) des Separators (158) erfasst.

9. Urinanalysevorrichtung (12) nach einem der Ansprüche 1 bis 8, wobei die Streifen entlang eines Kreises oder eines Kreisbogens um eine Drehachse (A) des Drehträgers (44) verteilt sind und optional die Streifen sich jeweils parallel zu der Achse (A) erstrecken.

10. Urinanalysevorrichtung (12) nach einem der Ansprüche 1 bis 9, wobei das Analysesystem umfasst:
- mindestens eine Lichtquelle (LED; 176, 178), die auf einer Seite des drehbaren Trägers (44) angeordnet ist, wobei die mindestens eine Lichtquelle Licht in Richtung des drehbaren Trägers (44) emittiert, und,
- einen Sensor (99), der auf der anderen Seite des drehbaren Trägers (44) angeordnet ist, wobei der Sensor (99) der mindestens einen Lichtquelle zugewandt ist, so dass er Licht erfasst, das durch den drehbaren Träger (44) hindurchgeht.

11. Urinanalysevorrichtung (12) nach einem der Ansprüche 1 bis 10, wobei ein Gehäuse (22) die Testanordnung (24) umschließt, wobei das Gehäuse so konfiguriert ist, dass es vollständig innerhalb einer Toilette (10) positioniert werden kann.

12. Urinanalysevorrichtung (12) nach einem der Ansprüche 1 bis 11, wobei der Drehhalter abnehmbar in dem Gehäuse (22) angeordnet ist.

13. Urinanalysevorrichtung (12) nach einem der Ansprüche 1 bis 12, wobei die Testanordnung (24) zusätzlich einen Urinpräsenzsensor (38) umfasst, wobei der Sensor beispielsweise ein Temperatursensor ist.

14. Urinanalysevorrichtung (12) nach einem der Ansprüche 1 bis 13, wobei der Injektor (46) in einem radial inneren Bereich der drehbaren Halterung (44) angeordnet ist.

15. Urinanalysevorrichtung (12) nach einem der Ansprüche 1 bis 14 in Kombination mit Anspruch 10, wobei der Sensor (99) so konfiguriert ist, dass er die Winkelposition der drehbaren Halterung durch Erfassen einer oder mehrerer Markierungen auf der drehbaren Halterung überwacht.

## Claims

1. A urinalysis device (12) for positioning inside a toilet comprising a test assembly (24), which comprises:
- at least one rotatable holder (44) comprising a plurality of test strips (56);
- an injector (46) configured to inject a controlled volume of urine onto at least one of the test strips;
- an analysis system (50) configured to detect a urine injection result on the test strip;
**characterized in that** the test strips (56) of the plurality of test strips (56) are fixed to the rotating support and **in that** the injector (46) comprises a movable syringe (80) configured to be moved towards or away from a test strip (56).

2. The urine analysis device (12) according to the preceding claim, in which the or each rotary support (44) is configured to rotate a test strip (56) and present it selectively in front of the injector (46) and/or in front of the analysis system (50).

3. The urinalysis device (12) according to any of the preceding claims, in which each rotating support (44) has housings (54) for receiving one or more test strips (56).

4. The urine analysis device (12) according to the preceding claim, in which the housings (54) are closed by at least one cover (72), for example transparent or translucent.

5. The urine analysis device (12) according to claim 3 or 4, wherein the rotary support (44) is cylindrical, and the housings (54) are arranged on an inner wall (44b) of the rotary support (44).

6. The urine analysis device (12) according to one of claims 3 to 5, in which the rotary support comprises a separator (158) having the housings (54), the separator (158) being made of a flexible material, preferably elastomer, and a receptacle (156) receiving the separator (158).

7. The urine analysis device (12) according to the preceding claim, wherein the receptacle (156) comprises an annular portion (162) and a cylindrical portion (164), radially external to the annular portion (162).

8. The urine analysis device (12) according to the preceding claim, in which the separator (158) comprises holes (160) opening into the housings (54), and the cylindrical portion (164) in contact with the separator (158) is transparent, so that the analysis system (50) detects an analysis result by light transmission through the cylindrical portion (164) and the holes (160) of the separator (158).

9. The urine analysis device (12) according to any one of claims 1 to 8, wherein the strips are distributed along a circle or arc of a circle about an axis (A) of rotation of the rotary support (44), and, optionally, the strips each extend parallel to the axis (A).

10. The urine analysis device (12) according to any one of claims 1 to 9, wherein the analysis system comprises :
- at least one light source (LED; 176, 178) arranged on one side of the rotating support (44), the at least one light source emitting light in the direction of the rotating support (44), and
- a sensor (99) arranged on the other side of the rotating support (44), the sensor (99) facing the at least one light source so as to detect light passing through the rotating support (44).

11. The urine analysis device (12) according to any one of claims 1 to 10, wherein a housing (22) encloses the test assembly (24), the housing being configured to be positioned entirely within a toilet (10).

12. The urine analysis device (12) according to any one of claims 1 to 11, in which the rotary support is removably arranged in the housing (22).

13. The urine analysis device (12) according to any one of claims 1 to 12, wherein the test assembly (24) further comprises a urine presence sensor (38), the sensor being for example a temperature sensor.

14. The urine analysis device (12) according to any one of claims 1 to 13, in which the injector (46) is arranged in a zone radially internal to the rotary support (44).

15. The urine analysis device (12) according to any one of claims 1 to 14, in combination with claim 10 wherein the sensor (99) is configured to control the angular position of the rotating support by detecting one or more markers on the rotating support.
